(19) Europäisches Patentamt
European Patent Office
Office européen des brevets

(11) Veröffentlichungsnummer: **0 279 239 B1**

(12) # EUROPÄISCHE PATENTSCHRIFT

(45) Veröffentlichungstag der Patentschrift: **11.11.92**

(51) Int. Cl.⁵: **C07D 277/56**, A01N 43/78, C07D 417/12, C07F 9/6539

(21) Anmeldenummer: **88101183.7**

(22) Anmeldetag: **27.01.88**

(54) **Mikrobizide Mittel.**

(30) Priorität: **30.01.87 CH 351/87**

(43) Veröffentlichungstag der Anmeldung:
**24.08.88 Patentblatt 88/34**

(45) Bekanntmachung des Hinweises auf die Patenterteilung:
**11.11.92 Patentblatt 92/46**

(84) Benannte Vertragsstaaten:
**AT BE CH DE ES FR GB IT LI NL**

(56) Entgegenhaltungen:
**EP-A- 0 027 018**
**EP-A- 0 044 201**
**EP-A- 0 064 353**
**GB-A- 2 020 662**
**US-A- 3 725 427**

(73) Patentinhaber: **CIBA-GEIGY AG**
**Klybeckstrasse 141**
**CH-4002 Basel(CH)**

(72) Erfinder: **Töpfl, Werner, Dr.**
**Dorneckstrasse 68**
**CH-4143 Dornach(CH)**
Erfinder: **Nyfeler, Robert, Dr.**
**Bärenfelserstrasse 8**
**CH-4057 Basel(CH)**
Erfinder: **Föry, Werner, Dr.**
**Inzlingerstrasse 11**
**CH-4125 Riehen(CH)**

(74) Vertreter: **Zumstein, Fritz, Dr. et al**
**Bräuhausstrasse 4**
**W-8000 München 2(DE)**

EP 0 279 239 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft die Verwendung als mikrobizide Wirkstoffe von 2-Chlor-4-trifluormethyl-thiazol-5-carbonsäurederivaten der nachstehenden Formel I, neue solche Derivate und sie enthaltende Mittel, sowie Verfahren zur Bekämpfung oder Verhütung des Befalls von Pflanzen durch phytopathogene Mikroorganismen.

Die Wirkstoffe entsprechen der Formel I

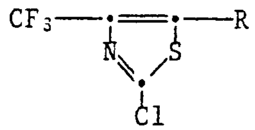

(I)

in welcher
R für einen organischen Rest mit maximal 40 Kohlenstoffatomen, der gegebenenfalls auch Stickstoff, Sauerstoff oder Schwefel enthalten kann und der sich durch Hydrolyse und/oder Oxydation in eine an den Thiazolring gebundene Carboxylgruppe umwandeln lässt, steht.

Thiazol-5-carbonsäurederivate sind aus der Literatur bekannt. Im US Patent 3 725 427 werden 2,4-dimethylthiazol-5-carboxamide als Fungizide beschrieben; in den US Patenten 4 199 506, 4 251 261 sowie in den Europäischen Offenlegungsschriften EP-A 27 018, 44 201 und 64 353 GB-A 2 020 662 sind 2-Chlor-4-trifluormethyl-thiazol-5-carbonsäurederivate als Gegenmittel (Antidots) zur Verminderung der phytotoxischen Wirkung von starken Herbiziden an Kulturpflanzen beschrieben worden.

Es hat sich nun gezeigt, dass die erfindungsgemässen 2-Chlor-4-trifluormethyl-thiazol-5-carbonsäurederivate der Formel I aussergewöhnlich gute mikrobizide Wirkung haben und Pflanzen vom Befall von phytopathologischen Mikroben und Pilzen zu schützen oder zu kurieren vermögen.

Die Verbindungen der Formel I sind bei Raumtemperatur stabil. Sie lassen sich auf dem Agrarsektor oder verwandten Gebieten insbesondere präventiv und kurativ zur Bekämpfung von pflanzenschädigenden Mikroorganismen einsetzen. Die Wirkstoffe der Formel I zeichnen sich in weiten Anwendungskonzentrationen durch eine sehr gute fungizide Wirkung und problemlose Anwendung aus. Darüber hinaus besitzen die Verbindungen nematizide Eigenschaften, wodurch sie auch zur Bekämpfung von Nematoden, insbesondere von pflanzenschädigenden Nematoden, geeignet sind.

Aufgrund ihrer ausgeprägten mikrobiziden Wirksamkeit sind diejenigen Verbindungen der Formel I bevorzugt, welche unter die Formel Ia fallen,

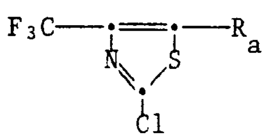

(Ia)

worin $R_a$ einen Rest Cyan, $-COXR_1$, $-CONR_3R_4$ oder -CO-D,
X Sauerstoff oder Schwefel,
$R_1$ Wasserstoff; $C_1$-$C_{18}$ Alkyl oder $C_3$-$C_{18}$ Cycloalkyl, welches unsubstituiert oder substituiert durch Halogen, eine Gruppe $-YR_2$, A, Nitro, $-N(R_3)COA$, $-[N(R_3)]_m-C(A)=NC_1$-$C_4$ Alkyl, $-[N(R_3)]_m-C(A)=NH$ oder $-[N(R_3)]_m-CO-N[(CO)_mR_3]-N(R_3)$, $-[N(R_3)]_m-CON[(CO)_m-R_3]-N(R_3)_m-(CO)_mR_4$, in welchem Rest einer der Indices m Null sein muss, ferner kann der $C_1$-$C_{18}$ Alkyl- oder $C_3$-$C_{18}$ Cycloalkylrest substituiert sein durch Cyano, einen Rest $-C(X)_m-XR_{10}$, $-XCXR_{10}$, $-(X)_m-CXA$, $-(X)CXN(R_3)N(R_3)R_4$, $-CHA-COOR_{10}$, $-C(OR_7)(OR_8)-R_9$, $-PO(R_5)R_6$, $C_3$-$C_8$-Cycloalkyl oder $C_5$-$C_8$ Cycloalkenyl; $R_1$ ist ferner $C_3$-$C_8$ Alkenyl oder $C_3$-$C_{18}$ Cycloalkenyl, welches unsubstituiert oder substituiert ist durch Halogen, $C_1$-$C_4$ Alkoxy, $C_1$-$C_4$ Alkylthio, $C_1$-$C_4$ Halogenalkoxy, $C_1$-$C_4$ Halogenalkyl oder einen Rest $-CO(O)_m-R_{10}$, -COA, $-CON(A)R_3$ oder $-PO(R_5)R_6$, $R_1$ ist ferner $C_3$-$C_8$ Alkinyl, welches unsubstituiert ist oder ein Rest $-(E)_mU$ oder $-(E)_m-Q$,
$R_2$ $C_1$-$C_8$Alkyl oder $C_3$-$C_8$ Cycloalkyl, welches unsubstituiert oder durch $C_1$-$C_8$Alkoxy, $C_1$-$C_8$ Alkylthio, $C_3$-$C_8$ Alkoxyalkoxy, Halogen, Cyan oder einen Rest $-CX(X)_mR_{10}$, $-(X)_m-CX-A$, $-(X)_mCXR_{10}$, $N(R_3)COA$, $-[N(R_3)]_m-C(A)=NH$, $-[N(R_3)]_m-C(A)=NC_1$-$C_4$Alkyl, A, -X-U oder -XQ substituiert ist, $R_2$ bedeutet ferner $C_3$-$C_8$Alkenyl oder $C_3$-$C_8$ Cycloalkenyl, welches unsubstituiert oder durch Halogen substituiert ist, oder einen Rest $-(E)_mU$ oder $-(E)_mQ$;
m ist Null oder 1;

Y ist Sauerstoff, Schwefel, SO oder $SO_2$,
A ist ein Rest $-N(R_3)R_4$,
D ist ein Rest $-N(R_3)N(R_4)(CO)_mR_3$,
$R_3$ und $R_4$ sind unabhängig voneinander je Wasserstoff, $C_1$-$C_8$Alkyl oder $C_3$-$C_8$Cycloalkyl, welches unsubstituiert oder durch $C_1$-$C_8$Alkoxy, $C_2$-$C_8$Alkoxyalkoxy, $C_1$-$C_8$Alkylthio, Cyano, einen Rest $-COOR_{10}$, $C_1$-$C_4$Alkylcarbamoyl, Di-$C_1$-$C_4$alkylcarbamoyl, $C_1$-$C_4$Alkylamino, Di-$C_1$-$C_4$Alkylamino, Piperidinocarbonyl, Pyrrolidinocarbonyl, Piperidino oder Pyrrolidino substituiert ist; $R_3$ und $R_4$ sind ferner $C_3$-$C_8$Alkenyl oder $C_3$-$C_8$Cycloalkenyl, welches unsubstituiert oder substituiert ist durch Halogen, $C_1$-$C_8$ Alkoxy, $C_3$-$C_8$Cycloalkenyl, oder einen Rest Cyan, $-COOR_{10}$, $C_1$-$C_4$Alkylcarbamoyl, Di-$C_1$-$C_4$alkylcarbamoyl, U, Pyrrolidinocarbamoyl oder Piperidinocarbamoyl substituiert ist, $R_3$ und $R_4$ sind ferner $C_3$-$C_8$Alkinyl, welches unsubstituiert oder durch U substituiert ist, oder $R_3$ und $R_4$ bedeuten einen Rest $-(E)_m$-U oder $-(E)_m$-Q,
$R_3$ und $R_4$ zusammen mit dem Stickstoffatom, an das sie gebunden sind, bilden auch einen gesättigten oder ungesättigten 5 - 9 gliedrigen Heterocyclus, der ein- oder mehrfach durch Sauerstoff, Schwefel, Stickstoff, -NH-, $-NC_1$-$C_4$Alkyl, -CO- oder $-C(OR_7)OR_8$- unterbrochen und durch Halogen, Cyan, $C_1$-$C_8$Alkoxy, Amino, $C_1$-$C_4$Alkylamino, Di-$C_1$-$C_4$-Alkylamino, oder einen Rest $-COOR_{10}$ substituiert sein kann;
$R_5$ und $R_6$ sind unabhängig voneinander je Hydroxy, $C_1$-$C_4$Alkyl oder $C_2$-$C_4$Alkoxy;
$R_7$ und $R_8$ sind unabhängig voneinander je $C_1$-$C_4$Alkyl oder
$R_7$ und $R_8$ bilden zusammen eine 2 - 4 gliedrige Alkylenkette,
$R_9$ und $R_{10}$ sind unabhängig voneinander je Wasserstoff, $C_1$-$C_3$Alkyl, $C_3$-$C_8$Cycloalkyl, $C_3$-$C_8$Alkenyl, $C_3$-$C_8$Cycloalkenyl, $C_3$-$C_8$Alkinyl, $C_2$-$C_8$Alkoxyalkyl, $C_4$-$C_8$Alkoxyalkoxyalkyl, $C_1$-$C_4$Halogenalkyl, $-(C_1$-$C_3Alkyl)_mU$, $-(C_1$-$C_3Alkyl)_mQ$, $C_1$-$C_4$Halogenalkoxy, $C_1$-$C_4$ Halogenalkoxy $C_1$-$C_4$alkyl;
U ist ein Phenyl- oder Naphthylrest, der unsubstituiert oder ein-oder mehrfach durch Halogen, $C_1$-$C_4$Alkyl, $-Y-C_1$-$C_4$Alkyl, $C_1$-$C_4$Halogenalkyl, $C_1$-$C_4$Halogenalkoxy, Cyano, Nitro, -COOH, $-COOR_7$, $-CONH_2$, $-CONHR_7$, $-CON(R_7)_2$, $SO_2NHR_7$, $SO_2N(R_7)_2$, Pyrrolidino, Piperidino, Pyrrolidinocarbonyl oder Piperidinocarbonyl substituiert ist.

E ist eine $C_1$-$C_8$Alkylen, $C_2$-$C_8$Alkenylenkette, die unsubstituiert oder durch Halogen, $C_1$-$C_4$Alkoxy, $C_1$-$C_4$Alkylthio, $C_1$-$C_4$ Halogenalkoxy oder einen Rest $-CO(O)_mR_{10}$, $(CO)_m$-A, $-(CO)_mQ$ substituiert und/oder durch ein Glied -CO- oder $-C(OR_1)OR_8$- unterbrochen ist,
Q ist ein gesättigter oder ungesättigter 5 - 12 gliedriger Heterocyclus, der 1 - 4 Heteroatome oder eine Sulfinyl- oder Sulfonylgruppe auch in Kombination mit 1 - 2 Heteroatomen enthalten kann, der durch eine oder zwei Carbonylgruppen unterbrochen und benzanneliert sein kann.

Gute mikrobizide Wirkung zeigen insbesondere auch die Verbindungen der Formel Ib

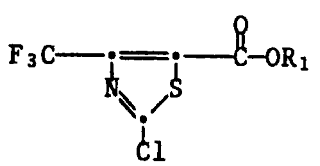

(Ib)

worin $R_1$ die oben gegebene Bedeutung hat.

Ebenfalls gute Wirkung zeigen die Verbindungen der Formel Ic

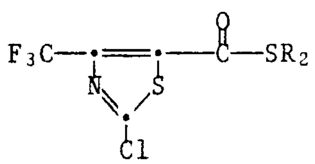

(Ic)

worin $R_2$ die oben gegebene Bedeutung hat, sowie die Verbindungen der Formel Id

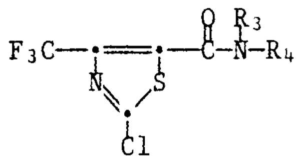

(Id)

worin $R_3$ und $R_4$ die oben gegebene Bedeutung haben.

Einige dieser 2-Chlor-4-trifluormethyl-thiazol-5-carbonsäurederivat-Wirkstoffe sind bekannt, grösstenteils handelt es sich jedoch um neue Verbindungen. Die neuen Verbindungen und ihre Herstellung bilden ebenfalls einen Gegenstand dieser Erfindung.

Neu sind die 2-Chlor-4-trifluormethyl-thiazol-5-carbonsäurederivate der Formel Ib

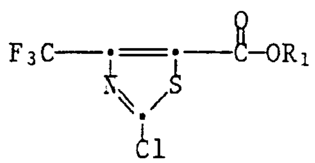

(Ib)

in denen $R_1$ einen Rest
Tetrahydropyran-2-ylmethyl-
2,2-Dimethyl-1,3-dioxolan-4-ylmethyl-
1,2-Dihydrobenz-1,4-dioxan-2-ylmethyl-
Thiophen-2-ylmethyl-
3,4 Methylendioxybenzyl-
5-Methylthiazol-4-ylethyl-
para Tolyl-eth-1-yl
Bornyl-
Norbornyl-
Fenchyl-
Menthyl-
Cyanoethyl-
Phenoxyethyl-
Methylsulfonylethyl
Phenylthioethyl-
Ethoxycarbonylmethyl-
Ethoxycarbonyleth-1-yl-
5,5-Diemthyl-tetrahydrofuran-2-on-3-yl-
2-Oxopyrrolinomethyl-
$\alpha$-Methoxycarbonyl-benzyl-
$\alpha$-Cyano-benzyl-
$\alpha$-Benzoyl-benzyl- oder
$\alpha$-Methoxycarbonyl-$\alpha$-phenyl-benzyl- oder den Morpholinomethylrest bedeutet.

Gute Wirkung zeigen auch die neuen Verbindungen der Formel Ib, in denen $R_1$ einen $C_1$-$C_{12}$Alkylrest bedeutet, der durch -$(X)_m$-CXA substituiert ist, wobei A, m und X die oben gegebene Bedeutung haben; besonders diejenigen, in denen m Null, X, Sauerstoff bedeuten; insbesondere diejenigen, in denen $R_1$ einen Azepinoethyl-, N-(2,6-Dimethylphenyl)-N-(methoxycarbonyleth-1-yl)-carbonyl-methyl- oder 2-Piperidino-eth-2-ylrest bedeutet.

Neu sind die 2-Chlor-4-trifluoromethyl-thiazol-5-thiocarbonsäurederivate der Formel Id

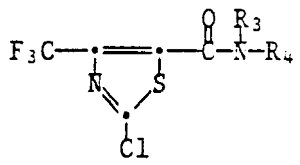

(Id)

worin eines von $R_3$ und $R_4$ Wasserstoff, $C_1$-$C_8$-Alkyl unsubstituiert, das andere oder beide $C_3$-$C_8$-Cycloalkyl, welches unsubstituiert ist, oder $C_1$-$C_8$-Alkyl oder $C_3$-$C_8$ Cycloalkyl, welches durch $C_1$-$C_8$-Alkoxy, $C_2$-$C_8$ Alkoxyalkoxy, $C_1$-$C_8$ Alkylthio, Cyano, einen Rest -$COOR_{10}$, $C_1$-$C_4$ Alkylcarbamoyl, Di-$C_1$-$C_4$ alkylcarbamoyl, $C_1$-$C_4$ Alkylamino, Di-$C_1$-$C_4$ Alkylamino, Piperidinocarbonyl, Pyrrolidinocarbonyl, Piperidino oder Pyrrolidino substituiert ist; $R_3$ und $R_4$ sind ferner $C_3$-$C_8$ Alkenyl oder $C_3$-$C_8$ Cycloalkenyl, welches unsubstituiert oder substituiert ist durch Halogen, $C_1$-$C_8$ Alkoxy, $C_3$-$C_8$ Cycloalkenyl, oder einen Rest Cyan, -$COOR_{10}$, $C_1$-$C_4$ Alkylcarbamoyl, Di-$C_1$-$C_4$ alkylcarbamoyl, U, Pyrrolidino-carbamoyl oder Piperidinocarbamoyl substituiert ist, $R_3$ und $R_4$ sind ferner $C_3$-$C_8$ Alkinyl, welches unsubstituiert oder durch U substituiert ist, oder $R_3$ und $R_4$ bedeuten einen Rest -$(E)_m$-U oder -$(E)_m$-Q, sind, und
$R_3$ und $R_4$ zusammen mit dem Stickstoffatom, an das sie gebunden sind, bilden auch einen gesättigten oder ungesättigten 5 - 9 gliedrigen Heterocyclus, der ein- oder mehrfach durch Sauerstoff, Schwefel, Stickstoff, -NH-, -$NC_1$-$C_4$ Alkyl, -CO- oder -$C(OR_7)OR_8$- unterbrochen und durch Halogen, Cyan, $C_1$-$C_8$ Alkoxy, Amino, $C_1$-$C_4$ Alkylamino, Di-$C_1$-$C_4$-Alkylamino, oder einen Rest -$COOR_{10}$ substituiert sein kann; wobei E, m, Q, $R_{10}$ und U die unter der Formel Ia gegebene Bedeutung haben.

Von diesen Amiden wirken diejenigen besonders gut, in denen
-$CON(R_3)R_4$ einen Rest
-N 2,4-Dichlorbenzyl-N-methylcarbamoyl-,
-N-Benzyl-N-isopropylcarbamoyl-,
-N-Cyclohexyl-N-methoxycarbamoylethyl-carbamoylamin-,
-N-2,6-Dimethylphenyl-N-methoxycarbamoyleth-1-yl-carbamoyl-
oder den -N-(1-Cyanopent-1-yl)-N-methyl-carbamoylrest bedeutet.

Neu sind weiter die 2-Chlor-4-trifluormethyl-thiazol-5-carbonsäurehydrazide der Formel Ie

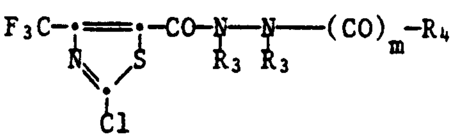

(Ie)

worin m, die $R_3$ und $R_4$ unabhängig voneinander eine unter der Formel Ia gegebene Bedeutung haben.

Darunter zeigten die Verbindungen der Formel If besonders gute Wirkung.

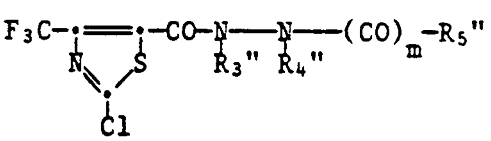

(If)

worin $R_3''$, $R_4''$ und $R_5''$ unabhängig voneinander je Wasserstoff, $C_1$-$C_8$-Alkyl, $C_3$-$C_8$-Cycloalkyl, Phenyl oder Benzyl bedeuten, wobei Phenyl und Benzyl unsubstituiert, oder durch Halogen, $C_1$-$C_4$-Alkyl, $C_1$-$C_4$-Haloalkyl, $C_1$-$C_4$-Alkoxy, $C_1$-$C_4$-Haloalkoxy, Nitro, Carboxyl, $C_1$-$C_4$-Alkoxyxcarbonyl, Carbamoyl, Methylcarbamoyl, substituiert ist oder $R_3''$ und $R_4''$ zusammen auch eine 4-5 gliedrige Alkylenkette bilden, die durch Sauerstoff, Schwefel, Imino oder $C_1$-$C_4$-Alkylimino unterbrochen sein kann und die ein- oder mehrmals durch $C_1$-$C_4$-Alkyl substituiert sein kann, bedeuten.

In diesen Definitionen werden unter Alkylresten Reste mit 1 bis 18 Kohlenstoffatomen verstanden. Diese Reste können geradkettig oder verzweigt sein. Die gebräuchlichsten Reste sind beispielsweise Methyl, Aethyl, n-Propyl, Isopropyl, n-Butyl, Isobutyl, sek.Butyl, tert.Butyl, n-Pentyl, Isopentyl, n-Hexyl und n-Octyl. Die Alkenyl- und Alkinylreste können ebenfalls geradkettig oder verzweigt sein und umfassen 3 bis 18

Kohlenstoffatome. Die am verbreitetsten Reste sind beispielsweise Allyl, Methallyl, Buten, Butadien, Propinyl, Methylpropinyl, 1-Butinyl, 2-Butinyl. Cycloalkyl- oder Cycloalkenylreste haben vorzugsweise 3 - 12 Kohlenstoffatome, sie können auch benzanneliert sein. Typische Vertreter sind beispielsweise Cyclopropyl, Cyclopentyl, Cyclohexyl, Cyclohexenyl, Indan, Tetrahydronaphthalin, Decalin. Unter Halogen wird Fluor, Chlor, Brom, Jod, insbesondere Fluor und Chlor verstanden. Halogenalkyl- und Halogenalkenyl-Reste können ein- oder mehrfach mit Halogen substituiert sein.

Die obgenannten Reste können unsubstituiert oder substituiert sein. Typische Substituenten dieser Reste sind beispielsweise die Halogenatome, über Sauerstoff, Schwefel oder eine Iminogruppe gebundene Alkyl-, Alkenyl-, Alkinyl-, oder Cycloalkyl-, Aryl- oder Aralkylreste, die ihrerseits wieder substituiert sein können. Diese Substituenten können aber auch über eine Sulfinyl-, Sulfonyl-, Carbonyl-, Carbonyloxy, Carbonylthio, Carbamoyl-, Sulfamoyl- oder eine Amino-oxybrücke an die alicyclischen Kohlenwasserstoffreste gebunden sein.

Der Substituent Q aber auch die Reste $R_3$ und $R_4$ können zusammen mit dem Stickstoffatom, an das sie gebunden sind, auch einen gesättigten oder ungesättigten 5 bis 12-gliedrigen Heterocyclus bilden, der noch ein, zwei oder drei weitere Heteroatome oder eine Sulfinyl- resp. Sulfonylgruppe enthält, durch ein oder zwei Carbonylgruppen unterbrochen sein kann, und welcher benzannelliert, unsubstituiert oder substituiert sein kann.

Als Heteroatome kommen dabei ein, zwei oder drei weitere Stickstoffatome, bis zwei Schwefel- oder Sauerstoffatome in Frage, wobei 2 Sauerstoffatome nicht direkt benachbart sein können

Beispiele für solche Heterocyclen sind untenstehend aufgeführt:

Pyrrolin, Pyrrolidin, Imidazolin, Imidazolidin, Pyrazolin, Pyrazolidin, Isoxazolin, Isoxazolidin, Oxazolin, Oxazolidin, Isothiazolidin, Thiazoline, Thiazolidine, Dithiazolidine, Oxadiazolidine, Piperidin, Piperazin, Tetrahydropyrimidin und -pyrazin, Morpholin, Thiomorpholin, Thiazine, Hexahydrotriazine, Tetrahydrotriazine, Oxadiazine, Oxatriazine, Hexahydroazepin, Hexahydrodiazepine, Diazepine, Hexahydrooxazepine, Azacyclooctan, Indolin, Isoindolin, Benzimidazolin, Benzindazolin, Benzoxazolin, Benzthiazoline, Benzisoxazolin, Benztriazol, Tetrahydrochinolin, Tetrahydroisochinolin, Tetrahydrochinazolin, Tetrahydrochinoxalin, Tetrahydrophtalazin, Benzmorpholin, Benzothiomorpholin, Tetrahydrobenzazepine und Tetrahydrodiazepine, Tetrahydrobenzoxazepine, 1,5-diazabicyclo[4,3,0]-nonane, Dihydrobenzoxazine, 1,6-diazabicyclo[5,3,0]decane, 1,4-diazabicyclo[3,3,0]octane, 1,5-diazabicyclo[4,4,0]decane.

Oben erwähnte Heterocyclen können auch die Bedeutung von Substituenten haben. Weitere Beispiele von heterocyclischen Systemen mit Substituentenfunktion sind beispielsweise Pyrrol, Imidazol, Pyrazol, Isoxazol, Oxazol, Isothiazol, Thiazol, Triazole, Oxadiazole, Thiadiazole, Tetrazole, Oxatriazole, Thiatriazole, Furan, Tetrahydrofuran, Dioxole, Dioxolane, Oxathiole, Oxathiolane, Thiophen, Tetrahydrothiophen, Dithiolane, Dithiazole, Pyridin, Pyrane, Thiopyrane, Pyridazin, Pyrimidin, Pyrazin, Tetrahydropyran, Tetrahydrothiopyran, Dioxine, Dioxane, Dithiine, Dithiane, Oxazine, Thiazine, Oxathiine, Oxathiane, Triazine, Oxadiazine, Thiadiazine, Oxathiazine, Dioxazine, Azepine, Oxepine, Thiepine, Diazepine, Oxazepine, Indole, Benzofurane, Benzothiophene, Indazole, Benzimidazole, Benzdioxole, Benzdithiole, Benzisoxazole, Benzthiazole, Benzoxazole, Benzoxathiole, Benztriazole, Benzoxadiazole, Benzofurazan, Benzothiadiazole, Chinolin, Isochinolin, Chromene, Chroman, Isochromen, Isochroman, Thiochromene, Isothiochromene, Thiochroman, Isothiochroman, Cinnolin, Chinazolin, Chinoxalin, Phtalazin, Benzdioxine, Benzdithiine, Benzoxazine, Benzdioxane, Benzoxathiane, Benzotriazine, Benzazepine, Benzdilazepine, Benzoxazepine, Purine, Pteridine, Phenoxazine, Phenothiazine.

Die heterocyclischen Reste können wie oben erwähnt substituiert sein.

Die Verbindungen der Formel I sind grösstenteils aus der Literatur bekannt oder können nach bekannten Methoden hergestellt werden.

Die Thiazol-5-carbonsäurederivate der Formel I werden z.B. gemäss USP 4,199,506 hergestellt, indem man einen Acrylsäureester der Formel II mit Chlorcarbonyl-sulfonylchlorid der Formel III umsetzt, entsprechend der Gleichung

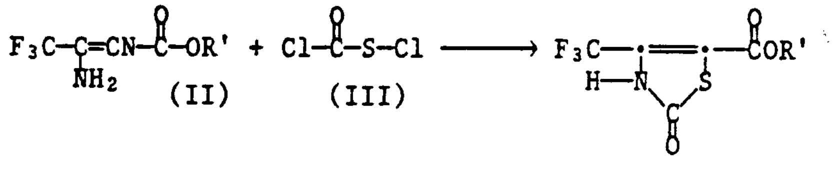

Das entstandene 2-Oxo-4-trifluormethyl-thiazol-5-carbonsäurederivat wird mit Phosphoroxychlorid behandelt, wobei entsprechend den Reaktionsbedingungen und der Menge Phosphoroxychlorid ein 2-Chlor-4-trifluormethyl-thiazol-5-carbonsäurederivat der Formel Ia oder 2-Chlor-4-trifluormethylthiazol-carbonsäurechlorid der Formel IIa entsteht:

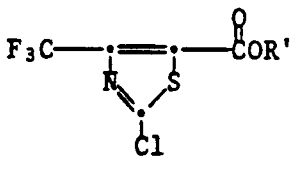

(Ia)

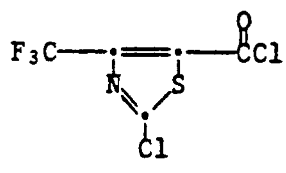

(IIa)

Die Acrylsäurederivate der Formel III können gemäss J. Het. Chem. 9 (1972) 513 hergestellt werden, indem man einen Acetoessigessigester mit Trifluormethylnitril umsetzt in Gegenwart von Natriumacetat in siedendem Lösungsmittel

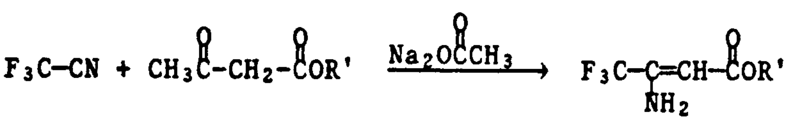

II

Ausgehend vom 2-Chlor-4-trifluormethylthiazol-5-carbonsäurechlorid können nach bekannten Methoden und entsprechend den folgenden Reaktionsschemen weitere Wirkstoffe der Formel I hergestellt werden:

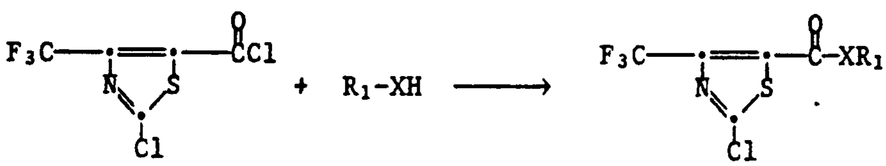

(IIa) (Ib)

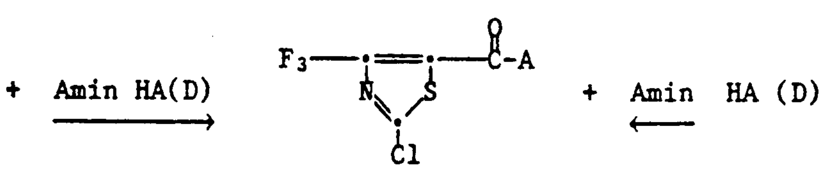


(Id)

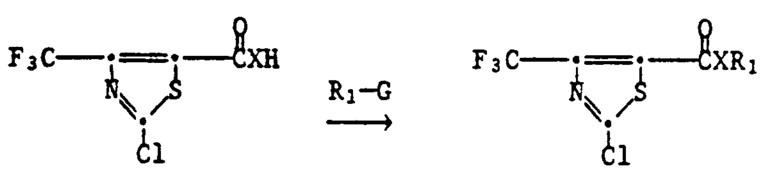


(IIa) (Ic)

In diesen Formeln haben A und $R_1$ die oben gegebene Bedeutung, während G eine nukleofuge Abgangsgruppe z.B. ein Halogenatom, ein aromatischer oder Niederalkyl-sulfoxyloxyrest bedeutet.

In diesen Umsetzungen werden in Anspassung an die jeweiligen Reaktionsbedingungen inerte Lösungs- und Verdünnungsmittel verwendet. Als Beispiele sind zu nennen:

Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Chlornaphthalin, Dichlornaphthalin, Tetrachlorkohlenstoff, Trichlorethan, Trichlorethylen, Pentachlorethan, Difluorbenzol, 1,2-Dichlorethan, 1,1-Dichlorethan, 1,2-cis-Dichlorethylen, Chlorbenzol, Fluorbenzol, Brombenzol, Jodbenzol, Dichlorbenzol, Dibrombenzol, Chlortoluol, Trichlorbenzol; Ether, wie Ethylpropylether, Methyl-tret.-butylether, n-Butylethylether, Di-n-butylether, Di-isobutylether, Diisoamylether, Diisopropylether, Anisol, Phenetol, Cyclohexylmethylether, Diethylether, Ethylenglykoldimethylether, Tetrahydrofuran, Dioxan, Thioanisol, Dichlordiethylether; Nitrokohlenwasserstoffe wie Nitromethan, Nitroethan, Nitrobenzol, Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, wie Heptan, Pinan, Nonan, Cymol, Benzinfraktionen innerhalb eines Siedepunktintervalles von 70° bis 190°C, Cyclohexan, Methylcyclohexan, Dekalin, Petrolether, Hexan, Ligroin, Trimethylpentan, Trimethylpentan, 2,3,3-Trimethylpentan, Octan; Ester wie Ethylacetat, Acetessigester, Isobutylacetat; Amide, z.B. Formamid, Methylformamid, Dimethylformamid.

Es wurde nun überraschend gefunden, dass die erfindungsgemässen Mittel, die Verbindungen der Formel I als Aktivstoffe enthalten, ein für praktische Bedürfnisse sehr günstiges Mikrobizid-Spektrum gegen phytopathogene Pilze und Bakterien aufweisen. Sie besitzen sehr vorteilhafte kurative, systemische und insbesondere präventive Eigenschaften und lassen sich zum Schutz von zahlreichen Kulturpflanzen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe der Formel I sind gegen die den folgenden Klassen angehörenden phytopathogenen Pilze wirksam: Fungi imperfecti (z.B. Botrytis, Helminthosporium, Fusarium Septoria, Cercospora und Alternaria); Basidiomyceten (z.B. die Gattungen Hemileia, Rhizoctonia, Puccinia); ferner wirken sie gegen die Klasse der Ascomyceten (z.B. Venturia, Podosphaera, Erysiphe, Monilinia, Uncinula). Ueberdies wirken die Verbindungen der Formel I systemisch. Sie können ferner als Beizmittel zur Behandlung von Saatgut (Früchte, Knollen, Körner) und Pflanzenstecklingen zum Schutz von Pilzinfektionen sowie gegen im Erdboden auftretende phytopathogene Pilze eingesetzt werden.

Neben der mikrobiziden Wirksamkeit weisen die Wirkstoffe der Formel I nematizide Eigenschaften auf, was sie insbesondere zur Bekämpfung von Pflanzennematoden geeignet macht. Zu diesem Zweck können die erfindungsgemässen Mittel kurativ, präventiv oder systemisch eingesetzt werden. Dabei entfalten sie eine breit gefächerte Aktivität gegen die verschiedenen Nematodenspecies und werden somit den Erfordernissen der Praxis gerecht.

Die erfindungsgemässen Wirkstoffe zeichnen sich durch besonders gute Pflanzenverträglichkeit aus.

Die Erfindung betrifft auch die Verwendung der Verbindungen der Formel I zur Bekämpfung phytopathogener Mikroorganismen, insbesondere pflanzenschädigender Pilze bzw. die präventive Verhütung eines Befalls von Pflanzen.

Darüber hinaus schliesst die vorliegende Erfindung auch die Herstellung agrochemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkulturen für die hierin offenbarten Indikationsgebiete gelten im Rahmen dieser Erfindung z.B. folgende Pflanzenarten: Getriede: (Weizen, Gerste, Roggen, Hafer, Reis, Sorghum und Verwandte); Rüben: (Zucker- und Futterrüben); Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Grapefruit, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Blumen, Sträucher, Laubbäume und Nadelbäume wie Koniferen). Diese Aufzählung stellt keine Limitierung dar.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden

oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen und Aufwandmenge richten sich dabei nach dem Befallsdruck für den entsprechenden Erreger (Pilzsorte). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt z.B. in Form von Granulat (Bodenapplikation). Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet. Darüber hinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg AS/ha, insbesondere bei 100 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon,Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden. Besonders vorteilhafte, applikationsfördernde Zuschlagstoffe, die zu einer starken Reduktion der Aufwandmenge führen können, sind ferner natürliche (tierische oder pflanzliche) oder synthetische Phospholipide aus der Reihe der Kephaline und Lecithine, die man beispielsweise aus Sojabohnen gewinnen kann. Verwendbare Handelsmischungen sind z.B. Phosphatidylchlorin-Mischungen. Synthetische Phospholipide sind z.B. Dioctanoylphosphatidylcholin und Dipalmitoylphosphatidylcholin.

Als oberflächenaktive Verbindungen kommen je nach Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze zu erwähnen.

Häufiger werden jedoch sog. synthetische Tenside verwendet, insbesondere Fettalkoholsulfonate, Fettalkoholsulfate, sulfonierte Benzimidazolderivate oder Alkylsulfonate.

Die Fettalkoholsulfonate oder -sulfate liegen in der Regel als Alkali-, Erdalkali- oder gegebenenfalls

substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die agrochemischen Zubereitungen enthalten in der Regel 0,1 bis 99 Gew.-%, insbesondere 0,1 bis 95 Gew.-%, Wirkstoff der Formel I, 99,9 bis 1 Gew.-% insbesondere 99,8 bis 5 Gew.-%, eines festen oder flüssigen Zusatzstoffes und 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die folgenden Beispiele dienen zur näheren Erläuterung der Erfindung ohne dieselbe einzuschränken. Temperaturen sind in den Beispielen und den nachfolgenden Tabellen in Centigraden gegeben, die Druckangaben sind in Millibar (mbar).

1. Herstellungsbeispiele

Beispiel 1.1

Herstellung von 2-Chlor-5-(2-phenoxyäthyloxycarbonyl)-4-trifluormethyl-thiazol

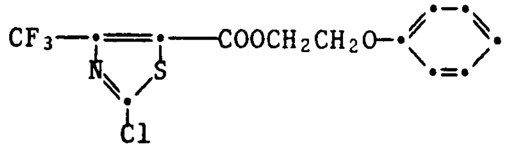

Zu einer Lösung von 11 g (0.044 Mol) 2-Chlor-5-chlorcarbonyl-4-trifluormethyl-thiazol in 100 ml absolutem Toluol gibt man unter Rühren 6.3 g (0.045 Mol) 2-Phenoxyethanol. Dann wird die Lösung gekühlt und bei 0 - 5° tropft man unter Rühren 3.6 g (0.045 Mol) Triäthylamin zu. Dabei fällt das Triäthylamin-Hydrochlorid aus. Nachdem alles zugegeben ist, wird die entstandene Suspension während 20 Stunden bei Raumtempeatur weitergerührt und dann auf Eiswasser gegossen. Die organische Phase wird abgetrennt, über Natriumsulfat getrocknet und im Rotationsverdampfer eingeengt. Der Rückstand kristallisiert und wird zwecks Reinigung in Petroläther verrührt, abgenutscht und getrocknet. Man erhält so 8.7 g Titelverbindung (57 % der Theorie). Schmelzpunkt 70 - 72 ° C.

Beispiel 1.2: Herstellung von 2-Chlor-5-(N-benzyl-N-isopropylamido)-4-trifluormethyl-thiazol

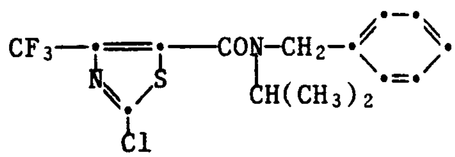

Zu einer Lösung von 6.5 g (0.025 Mol) 2-Chlor-3-chlorocarbonyl-4-trifluormethyl-thiazol in 100 ml trockenem Aethylacetat tropft man unter Rühren und Kühlen bei 0 - 5 ° eine Lösung von 2,8 g (0,027 Mol) Triäthylamin und 4.1 g N-Isopropylbenzylamin (0.027 Mol) in 50 ml Aethylacetat. Nachdem alles zugegeben ist, wird die entstandene gelbe Lösung während 15 Stunden bei Raumtemperatur weitergerührt, dann auf Eiswasser gegossen. Die organische Phase wird abgetrennt über Natriumsulfat getrocknet mit Aktivkohle gereinigt, filtriert und am Rotationsverdampfer eingeengt. Als Rückstand bleibt ein rotbraunes Oel, das zur Reinigung mit Essigester/Hexan über eine Kieselgelsäule chromatographiert wird. Nach Verdampfen des Laufmittels verbleiben 6.5 g Titelprodukt als farbloses analysenreines Oel (73% der Theorie).

Beispiel 1.3: Herstellung von 2-Chlor 5-(1-methoxycarbonyl-äth-1-yloxy-carbonyl)-4-trifluormethyl-thiazol

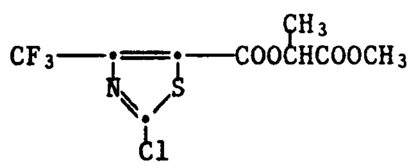

Ein Gemisch, bestehend aus 6,94 g 2-Chlor-5-carboxyl-4-trifluormethyl-thiazol, 5 g 2-Brompropionsäuremethylester und 4.5 g Kaliumcarbonat suspendiert in 50 ml wasserfreiem Dimethylformamid wird bei Raumtempeatur während 3 Stunden unter Stickstoffatmosphäre gerührt. Dann wird das Reaktionsgemisch filtriert und das Filtrat auf Eiswasser/Aethylacetat 1:1 gegossen. Die organische Phase wird abgetrennt, 3x mit Eis-Wasser gewaschen, getrocknet und am Rotationsverdampfer eingeengt. Der Rückstand verbleibt als farbloses Oel, welches zur Reinigung destilliert wird. 8.5 g Titelprodukt (75 % der Theorie). Kp. 75 - 80 ° /0.015 mbar.

Auf analoge Weise werden folgende Verbindungen hergestellt:

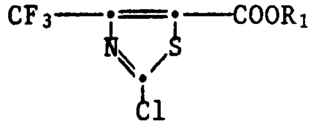

Tabelle 1

| No. | $R^1$ | phys. Konstante |
|---|---|---|
| 1.001 | 1-Dodecyl | |
| 1.002 | 1-Octadecyl | |
| 1.003 | Cyclopropylmethyl | |
| 1.004 | Cyclopentylmethyl | |
| 1.005 | Cyclohexylmethyl | |
| 1.006 | Cyclohexylethyl | |
| 1.007 | Tetrahydrofuran-2-yl-methyl | |
| 1.008 | Pentahydropyran-2-yl-methyl | Smp. 60 - 63° |
| 1.009 | 2,2-Dimethyl-1,3-dioxolan-4-yl-methyl | $n_D^{20}$ 1.4804 |
| 1.010 | 1,2-Dihydrobenz-1,4-dioxan-2-ylmethyl | Harz |
| 1.011 | Furan-2-ylmethyl | |
| 1.012 | Thiophen-2-ylmethyl | $n_D^{20}$ 1.5410 |
| 1.013 | 3,4-Methylendioxybenzyl | Smp. 96 - 98° |
| 1.014 | Thiophen-2-ethyl | |
| 1.015 | 5-Methyl-thiazol-4-yläthyl | |
| 1.016 | Phenylethyl | |
| 1.017 | para Tolyl-eth-1-yl | Oel |
| 1.018 | 3,45-Trimethoxybenzyl | |
| 1.019 | Geranyl | |
| 1.020 | 2-Hexen-1-yl | |
| 1.021 | 1-Hexen-6-yl | |
| 1.022 | 1-Hexen-3-yl | |
| 1.023 | 2-Chlor-2-propen-1-yl | |
| 1.024 | 3-Chlor-2-propen-1-yl | |
| 1.025 | 3-Phenyl-2-propen-1-yl | |
| 1.026 | Cyclopentyl | |
| 1.027 | Cyclohexyl | |
| 1.028 | 2-Methyl-cyclohexyl | |
| 1.029 | 3-Methyl-cyclohexyl | |
| 1.030 | 4-Methyl-cyclohexyl | |
| 1.031 | Cyclododecyl | |
| 1.032 | 2,3-Dimethyl-cyclohexyl | |
| 1.033 | 2,4-Dimethyl-cyclohexyl | |
| 1.034 | 2,6-Dimethyl-cyclohexyl | |
| 1.035 | 3,5-Dimethyl-cyclohexyl | |
| 1.036 | 4-Tert.butyl-cyclohexyl | |
| 1.037 | Bornyl | Smp. 65 - 67° |
| 1.038 | Norbornyl | $n_D^{20}$ 1.5026 |
| 1.039 | Fenchyl | Oel |
| 1.040 | Menthyl | $n_D^{20}$ 1.4860 |
| 1.041 | 2,2-Dichlor-cyclopropylmethyl | |
| 1.042 | $-CH_2-CN$ | |
| 1.043 | $-CH_2-CH_2-CN$ | $n_D^{20}$ 1.4941 |

| No. | $R^1$ | phys. Konstante |
|---|---|---|
| 1.044 | $-CH_2-PO(OC_2H_5)_2$ | |
| 1.045 | 2-Nitro-ethyl | |
| 1.046 | 2-Allyloxy-äthyl | |
| 1.047 | 2-Benzyloxy-äthyl | |
| 1.048 | para-Chlorbenzyloxyethyl | |
| 1.049 | ortho-Chlorbenzyloxyethyl | |
| 1.050 | Cyclopropyloxyethyl | |
| 1.051 | Cyclohexyloxyethyl | |
| 1.052 | 2-Phenoxy-ethyl | Smp. 70 - 72° |
| 1.053 | para-Chlorphenoxyethyl | |
| 1.054 | $-CH_2-CH_2-SCH_3$ | |
| 1.055 | $-CH_2-CH_2-SO-CH_3$ | |
| 1.056 | $-CH_2-CH_2-SO_2-CH_3$ | Smp. 86 - 88° |
| 1.057 | $-CH_2-CH_2-CH_2-SCH_3$ | |
| 1.058 | $-CH_2-CH_2-CH_2-SO-CH_3$ | |
| 1.059 | $-CH_2-CH_2-CH_2-SO_2-CH_3$ | |
| 1.060 | $-CH_2-CH_2-S-C_4H_9$(") | |
| 1.061 | $-CH_2-CH_2-S-CH_2-CH=CH_2$ | |
| 1.062 | Cyclohexylthioethyl | |
| 1.063 | Benzylthioethyl | |
| 1.064 | para-Chlor-benzylthioethyl | |
| 1.065 | Phenylthioethyl | Smp. 47 - 49° |
| 1.066 | Phenylsulfonylethyl | |
| 1.067 | para-Tolylthioethyl | |
| 1.068 | para-Chlorphenylthioethyl | |
| 1.069 | Phenylthiopropyl | |
| 1.070 | β-Naphthylthioethyl | |
| 1.071 | 2-Phenylthio-1-methyl-ethyl | |
| 1.072 | 2-Phenylthio-1-chlormethyl-ethyl | |
| 1.073 | $-CH_2-CH_2-S-CH_2-COOC_2H_5$ | |
| 1.074 | $-CH_2CH_2SCH(CH_3)COOC_2H_5$ | |
| 1.075 | $-CH_2-CH_2-S-CO-N(CH_3)_2$ | |
| 1.076 | Piperidinoylthioethyl | |
| 1.077 | $-CH_2-CH_2-S-CS-N(CH_3)_2$ | |
| 1.078 | Piperidinothiocarbonylthioethyl | |
| 1.079 | $-CH_2-COOCH_3$ | |
| 1.080 | $-CH_2-COOC_2H_5$ | Smp. 80 - 85°/ 0.025 mm |
| 1.081 | $-CH_2-COOC_4H_9(n)$ | |
| 1.082 | $-CH(CH_3)COOCH_3$ | Smp. 25 - 80°/ 0.015 mm |
| 1.083 | $-C(CH_3)_2COOC_2H_5$ | |
| 1.084 | $-CH_2-CH_2-COOC_2H_5$ | |
| 1.085 | 5,5-Dimethyl-tetrahydrofuran-2-on-3-yl | Smp. 100 - 102° |
| 1.086 | $-CH_2-CO-N(C_2H_5)_2$ | |
| 1.087 | $-CH_2CON[CH(CH_3)_2]_2$ | |
| 1.088 | $-CH_2CON[CH_2(CH_3)C_2H_5]_2$ | |
| 1.089 | $-CH_2CON(CH_2CH=CH_2)_2$ | |
| 1.090 | 2-Methylpiperidinoylmethyl | |
| 1.091 | Azepinoylmethyl | $n_D^{20}$ 1.5172 |

| No. | $R^1$ | phys. Konstante |
|---|---|---|
| 1.092 | Anilidomethyl | |
| 1.093 | N-Methyl-anilidomethyl | |
| 1.094 | N-(2,6-Dimethylphenyl)-N-(methoxy-carbonyl-eth-1-yl)-carbamoylmethyl | Smp. 95 - 97° |
| 1.095 | 1-(Piperidinocarbonyl)-eth-1-yl | Smp. 62 - 66° |
| 1.096 | $-CH_2-CH_2-NH-CO-CH_3$ | |
| 1.097 | Cyclopropan-amoyl-ethyl | |
| 1.098 | $-CH_2-CH_2-NH-CO-CH_2-Cl$ | |
| 1.099 | $-CH_2-CH_2-NH-CO-CHCl_2$ | |
| 1.100 | Benzamoyl-ethyl | |
| 1.101 | Thienyl-2-carbamoylethyl | |
| 1.102 | Furfuramoylethyl | |
| 1.103 | $-CH_2-CH_2-NH-CO-NH-CH_3$ | |
| 1.104 | $C(CH_3)_3$ | |
| 1.105 | Phenylureylene-ethyl | |
| 1.106 | $-CH_2CH_2N(CH_3)COCH_3$ | |
| 1.107 | $-CH_2CH_2N(CH_3)COCHCl_2$ | |
| 1.108 | $-CH_2CH_2N(CH_3)CONHCH_3$ | |
| 1.109 | $-CH_2CH_2N(CH_2)SO_2CH_3$ | |
| 1.110 | N-Methyl-phenylsulfamoyl-ethyl | |
| 1.111 | $-CH_2CH_2N(C_3H_7-i)COCHCl_2$ | |
| 1.112 | $-CH_2CH_2N(CH_2CH{=}OH_2)CONHCl_2$ | |
| 1.113 | 2-Oxo-pyrrolidino-ethyl | Smp. 58 - 62° |
| 1.114 | Dicyclohexylmethyl | |
| 1.115 | α-Phenylbenzyl | $n_D^{20}$ 1.5695 |
| 1.116 | α-Methylbenzyl | |
| 1.117 | α-Carboxylbenzyl | |
| 1.118 | α-Carboxyl-para-chlorbenzyl | |
| 1.119 | α-Methoxycarbonyl-benzyl | Smp. 62 - 64° |
| 1.120 | α-Ethoxycarbonyl-benzyl | |
| 1.121 | α-Cyanobenzyl | Smp. 78 - 81° |
| 1.122 | α-Benzoyl-benzyl | Smp. 105 - 109° |
| 1.123 | α-Methoxycarbonyl-α-phenylbenzyl | Smp. 106 - 110° |
| 1.124 | $-CH_2-CH_2-N(CH_3)_2$ | |
| 1.125 | Pyrrolidinoethyl | |
| 1.126 | Piperidinoethyl | |
| 1.127 | Morpholinoethyl | Smp. 185 - 187° (Hydrochlorid) |
| 1.128 | Anilinoethyl | |
| 1.129 | para (1-Methoxycarbonyl)ethoxyphenyl | |
| 1.130 | para(3-Methyl-1,3-oxazolidin-2-yl)phenyl | |
| 1.131 | para(N'N'-(Dimethyl-ureylene)-phenyl | |
| 1.132 | meta(N',N'-Dimethyl-ureylene)phenyl | |
| 1.133 | β-Cyano-β-Methoxycarbonyl-styryl-4-yl | |
| 1.134 | β,β-Di(Methoxycarbonyl)-styryl-4-yl | |
| 1.135 | β,β-Dicyano-styryl-4-yl | |

| Nr. | $R^1$ | | phys. Konstante |
|---|---|---|---|
| 1.136 | $-COOC_2H_5$ | | Smp. 59 - 60° |
| 1.137 | -COOH | | Smp. 122 - 125° |
| 1.138 | -COO Benzyl | | Smp. 56 - 58° |
| 1.139 | -COO(4,4-Dimethyl-tetrahydro-fur-3-yl-2-on) | | Smp. 100 - 102° |
| 1.140 | -COO(4-Methyl-thiazol-5-ylethyl) | | Smp. 64 - 67° |
| 1.141 | -COO(2,3,5,6-Diepoxy-cyclo-hexan-1-yl) | syn. isomer | Smp. 144 - 175° |
| 1.142 | -COO(2,3,5,6-Diepoxy-cyclo-hexan-1-yl) | anti isomer | Smp. 144 - 120° |
| 1.143 | α-(4-Chlorphenyl)benzyl | | |
| 1.144 | α-(2-Chlorphenyl)benzyl | | |
| 1.145 | α-(4-Chlorphenyl)-4-chlorbenzyl | | |
| 1.146 | α-(2-Chlorphenyl)-2-chlorbenzyl | | |
| 1.147 | α-(2-Chlorphenyl)-4-chlorbenzyl | | |
| 1.148 | α-(4-Fluorphenyl)-benzyl | | |
| 1.149 | α-(2-Fluorphenyl)-benzyl | | |
| 1.150 | α-(4-Fluorphenyl)-4-fluorbenzyl | | |
| 1.151 | α-(2-Fluorphenyl)-2-fluorbenzyl | | |
| 1.152 | α-(2-Fluorphenyl)-4-fluorbenzyl | | |
| 1.153 | α-(4-Tolyl)-benzyl | | |
| 1.154 | α-(4-Anisyl)-benzyl | | |
| 1.155 | α-(4-Methoxyphenyl)-benzyl | | |
| 1.156 | α-(3-Trifluorphenyl)-benzyl | | |

Tabelle 2

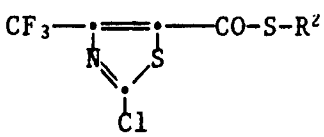

| Nr. | $R^2$ | Physikal. Daten |
|---|---|---|
| 2.001 | $-CH_2-COOH$ | |
| 2.002 | $-CH_2-COOCH_3$ | |
| 2.003 | $-CH_2-COOC_2H_5$ | |
| 2.004 | $-CH(CH_3)COOH$ | |
| 2.005 | $CH(CH_3)COOC_2H_5$ | |
| 2.006 | $-CH_2-CH_2-COOH$ | |
| 2.007 | $-CH_2-CO-N(C_2H_5)_2$ | |
| 2.008 | Piperidinamoylmethyl | |
| 2.009 | Azepinamoylmethyl | |
| 2.010 | Anilidomethyl | |
| 2.011 | para Chloranilidmethyl | |
| 2.012 | 2-Carboxylphenylmethyl | |
| 2.013 | Benzyl | Smp. 64 - 66° |

Tabelle 3

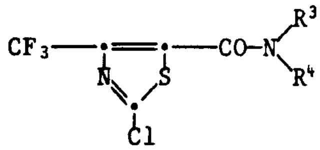

(Id)

| Nr. | $R^3$ | $R^4$ | Physikal.Daten |
|---|---|---|---|
| 3.001 | Allyl | Allyl | Smp. 38 - 40° |
| 3.002 | Allyl | 2-Methoxy-äthyl | |
| 3.003 | Allyl | Isopropyl | |
| 3.004 | 2-Methyl-2-propen-1-yl | Isopropyl | |
| 3.005 | 2-Methyl-2-propen-1-yl | Cyclohexyl | |
| 3.006 | 2-Chlor-2-propen-1-yl | 2-Methoxy-äthyl | |
| 3.007 | 2-Chlor-2-propen-1-yl | Isopropyl | |
| 3.008 | 2-Chlor-2-propen-1-yl | Cyclohexyl | Smp. 70 - 72° |
| 3.009 | 2-Chlor-2-propen-1-yl | 2-Chlor-2-propen-1-yl | Smp. 38 - 40° |
| 3.010 | 3-Chlor-2-propen-1-yl | Propyl | |
| 3.011 | Propyl | 2-Methoxy-äthyl | |
| 3.012 | Methyl | Cyclohexyl | |
| 3.013 | Methyl | Benzyl | $n_D^{20}$ 1.5408 |
| 3.014 | Methyl | 2,6-Dichlor-benzyl | Smp. 118 - 120° |
| 3.015 | Isopropyl | Benzyl | $n_D^{20}$ 1.5333 |
| 3.016 | Isopropyl | 4-Chlor-benzyl | |
| 3.017 | H | Benzyl | Smp. 102-105° |
| 3.018 | H | Phenyl | |
| 3.019 | Methyl | Phenyl | Smp. 57 - 59° |
| 3.020 | Methyl | 4-Chlor-phenyl | |
| 3.021 | Ethyl | 2-Chlor-4-brom-phenyl | |
| 3.022 | Ethyl | 3-Trifluormethyl-phenyl | |
| 3.023 | H | $-CH_2-COOC_2H_5$ | |
| 3.024 | Methyl | $-CH_2-COOCH_3$ | |
| 3.025 | Isopropyl | $-CH_2-CO-NH-C_3H_7(i)$ | |
| 3.026 | Cyclopropyl | $-CH_2-CO-N(C_2H_5)_2$ | |
| 3.027 | Ethyl | $-CH_2-CH_2-CN$ | |
| 3.028 | Allyl | $-CH_2-CH_2-CN$ | |
| 3.029 | Cyclohexyl | $-CH_2-CH_2-CN$ | |
| 3.030 | Phenyl | $-CH_2-CH_2-CN$ | |
| 3.031 | Phenyl | $-CH_2-CH_2-COOH$ | |
| 3.032 | Cyclohexyl | $-CH_2-CH_2-COOCH_3$ | Smp. 116 - 120° |
| 3.033 | 2,6-Dimethyl-phenyl | $-CH(CH_3)COOCH_3$ | Smp. 120 - 123° |
| 3.034 | Phenyl | $-CH_2-COOH$ | |
| 3.035 | Phenyl | $-CH_2-CN$ | |
| 3.036 | 4-Chlor-phenyl | $-CH_2-CN$ | |
| 3.037 | 2,4-Dichlor-phenyl | $-CH_2-CN$ | |
| 3.038 | 3,4-Dichlor-phenyl | $-CH_2-CN$ | Smp. 128 - 131° |
| 3.039 | 3-Trifluormethyl-phenyl | $-CH_2-CN$ | |
| 3.040 | Methyl | 1-Cyanocyclopent-1-yl | Smp. 130 - 133° |
| 3.041 | H | 3-Trifluoromethyl-cyclohexyl | |
| 3.042 | H | $-NH_2$ | |
| 3.043 | H | $-N(CH_3)_2$ | |
| 3.044 | Methyl | $-NH-CH_3$ | |

| Nr. | $R^3$ | $R^4$ | Physikal.Daten |
|---|---|---|---|
| 3.045 | H | Anilino | Smp. 137 - 138° |
| 3.046 | H | 2-Benzoyl-hydrazo | |
| 3.047 | H | 2-Phenylsulfonyl-hydrazo | |
| 3.048 | Pyrrolidino | | |
| 3.049 | Piperidino | | |
| 3.050 | 2-Methylpiperidino | | |
| 3.051 | 2-Ethylpiperidino | | |
| 3.052 | Hexahydroazepino | | |
| 3.053 | Morpholino | | |
| 3.054 | 2,2,5,5-Tetramethyl 1,3-oxazolidin-3-yl | | |
| 3.055 | 5,5-Dimethyl-2,2-tetra-methylen-1,3-oxazolidin-3-yl | | |
| 3.056 | 5,5-Dimethyl-2,2-pentamethylen-1,3-oxazolidin-3-yl | | |
| 3.057 | 2-Phenyl-1,3-oxazolidin-3-yl | | |
| 3.058 | 2,2-Tetramethylen-benzthiazol-3-yl | | |
| 3.059 | 2-Oxo-pyrrolidino | | |
| 3.060 | Hexahydro-2-oxo-azepino | | |
| 3.061 | 3-Oxo-thiomorpholino | | |
| 3.062 | 2-Oxo-1,3-oxazolin-3-yl | | |
| 3.063 | 2-Trichloromethyl-1,3-oxazolidin-3-yl | | |
| 3.064 | H | 2-Chlorbenzyl | |
| 3.065 | H | 2-Hexylbenzyl | |
| 3.066 | $C_2H_5$ | $C_2H_5$ | Smp. 40 - 41° |
| 3.067 | Allyl | H | Smp. 56 - 58° |
| 3.068 | Phenylethyl | H | Smp. 88 - 84° |
| 3.069 | $C_2H_5$ | 2,6-Dichlorbenzyl | Smp. 89 - 91° |
| 3.070 | 2-Chlorbenzyl | H | Smp. 115 - 116° |
| 3.071 | Allyl | Allyl | Smp. 100 - 101° |
| 3.072 | $C_4H_9$-n | 2,6-Dichlorbenzyl | $n_D^{20}$ 1.5491 |
| 3.073 | Allyl | Ethoxycroton-2-yl | Smp. 72 - 74° |
| 3.074 | $CH(CH_3)_2$ | 2-Chlorallyl | $n_D^{20}$ 1.5027 |
| 3.075 | 4-Chlor-2-fluor-6-iso-propoxyphenyl | H | Smp. 123 - 125° |
| 3.076 | Cyano-dimethylmethyl | Methoxyethyl | Smp. 130 - 132° |
| 3.077 | Chlorphenyl | H | Smp. 125 - 128° |
| 3.078 | Cyano-dimethyl-methyl | H | Smp. 78 - 80° |
| 3.079 | 2,2-Dimethylindanyl | H | Smp. 174 - 175° |
| 3.080 | 3,5-Bistrifluormethyl-phenyl | H | Smp. 125 - 127° |
| 3.081 | Diphenylmethyl | H | Smp. 177 - 179° |
| 3.082 | 2,6-Difluorphenyl | H | Smp. 160 - 161° |
| 3.083 | 5-Trifluormethyl-thiazol-2-yl | H | Smp. 136 - 138° |
| 3.084 | 2-Carboxyl-4-chlorphenyl | H | Smp. 132 - 134° |
| 3.085 | 3-Trifluormethylcyclo-hexyl | H | Smp. 106 - 109° |
| 3.086 | 2,4,6-Trichloranilino | H | Smp. 184 - 186° |
| 3.087 | Furfuryl | H | Smp. 100 - 102° |
| 3.088 | 3,4-Methylendioxybenzyl | H | Smp. 129 - 131° |
| 3.089 | 4-Amidosulfonylphenyl | H | Smp. 186 - 189° |
| 3.090 | 1,2-Diphenyleth-1-yl | H | Smp. 146 - 148° |

| Nr. | $R^3$ | $R^4$ | Physikal.Daten |
|---|---|---|---|
| 3.091 | α-Methylbenzyl | H | Smp. 131 - 133° |
| 3.092 | Benzoylamido | H | Smp. 192 - 194° |
| 3.093 | 4-Fluorbenzyl | H | Smp. 128 - 130° |
| 3.094 | 2,2-Diphenyleth-1-yl | H | Smp. 127 - 129° |
| 3.095 | 1-Cyano-cyclopent-1-yl | Methoxycarbonylmethyl | Smp. 145 - 147° |
| 3.096 | 1-Cyano-cyclohex-1-yl | H | Smp. 145 - 147° |
| 3.097 | 2-Methoxycarbonyl-4-chlorphenyl | H | |
| 3.098 | 2-Methylaminocarbonyl-4-chlorphenyl | H | |
| 3.099 | 2-Dimethylamino | H | |
| 3.100 | Anilino | H | |
| 3.101 | 2-Chloranilino | H | |
| 3.102 | 4-Chloranilino | H | |
| 3.103 | 1-Cyanocyclopent-1-yl | $CH_3$ | |

2. Formulierungsbeispiele für feste Wirkstoffe der Formel I (% = Gewichtsprozent)

| 2.1 Spritzpulver | a) | b) | c) |
|---|---|---|---|
| Wirkstoff aus den Tabellen 1 - 3 | 25 % | 50 % | 75 % |
| Na-Ligninsulfonat | 5 % | 5 % | |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.2 Emulsions-Konzentrat | |
|---|---|
| Wirkstoff aus den Tabellen 1 - 3 | 10 % |
| Ocrylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 2.3 Stäubemittel | a) | b) |
|---|---|---|
| Wirkstoff aus den Tabellen 1 - 3 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

| 2.4 Extruder Granulat | |
|---|---|
| Wirkstoff aus den Tabellen 1 - 3 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.5 Umhüllungs-Granulat | |
|---|---|
| Wirkstoff aus den Tabellen 1 - 3 | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| 2.6 Suspensions-Konzentrat | |
|---|---|
| Wirkstoff aus den Tabellen 1 - 3 | 40 % |
| Ethylenglykol | 10 % |
| Nonylphenolpolyethylenglykolether (15 Mol Ethylenoxid) | 6 % |
| N-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

3. Biologische Beispiele:

Beispiel 3.1: Wirkung gegen Puccinia graminis auf Weizen

a) Residual-protektive Wirkung

Weizenpflanzen werden 6 Tage nach der Aussaat mit einer aus Spritzpulver des Wirkstoffes hergestellten Spritzbrühe (0,006 % Aktivsubstanz) besprüht. Nach 24 Stunden werden die behandelten Pflanzen mit einer Uredosporensuspension des Pilzes infiziert. Nach einer Inkubation während 48 Stunden bei 95 - 100 % relativer Luftfeuchtigkeit und ca. 20 °C werden die infizierten Pflanzen in einem Gewächshaus bei ca.

22° C aufgestellt. Die Beurteilung der Rostpustelnentwicklung erfolgt 12 Tage nach der Infektion.

b) Systemische Wirkung

Zu Weizenpflanzen wird 5 Tage nach der Aussaat eine aus Spritzpulver des Wirkstoffes hergestellte Spritzbrühe gegossen (0,002 % Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95 - 100 % relativer Luftfeuchtigkeit und ca. 20° C werden die infizierten Pflanzen in einem Gewächshaus bei ca. 22° C aufgestellt. Die Beurteilung der Rostpustelnentwicklung erfolgt 12 Tage nach der Infektion.

Die geprüften Verbindungen aus den Tabellen 1 - 3 insbesondere 3.040 zeigen gegen Puccinia-Pilze gute Wirksamkeit. Sie verhindern den Puccinia-Befall fast vollständig (Befall = 0 - 10 %). Unbehandelte aber infizierte Kontrollpflanzen weisen dagegen einen Puccinia-Befall von 100 % auf.

Beispiel 3.2: Wirkung gegen Cercospora arachidicola auf Erdnusspflanzen

Residual-protektive Wirkung

10 - 15 cm hohe Erdnusspflanzen werden mit einer aus Spritzpulver der Wirksubstanz hergestellten Spritzbrühe (0,02 % Aktivsubstanz) besprüht und 48 Stunden später mit einer Konidiensuspension des Pilzes infiziert. Die infizierten Pflanzen werden während 72 Stunden bei ca. 21° C und hoher Luftfeuchtigkeit inkubiert und anschliessed bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100 %), zeigten Erdnusspflanzen, die mit Wirkstoffen aus den Tabellen 1 - 3 behandelt wurden, einen stark reduzierten Cercospora-Befall. Mit einzelnen Verbindungen konnte das Auftreten von Flecken fast vollständig verhindert werden (Befall = 0 - 10 %). Sehr gute Wirkung wurde mit Verbindung 1.009 erreicht.

Beispiel 3.3: Wirkung gegen Erysiphe graminis auf Gerste

a) Residual-protektive Wirkung

Ca. 8 cm hohe Gerstenpflanzen werden mit einer aus Spritzpulver des Wirkstoffes hergestellten Spritzbrühe (0,02 % Aktivsubstanz) besprüht. Nach 3-4 Stunden werden die behandelten Pflanzen mit Konidien des Pilzes bestäubt. Die infizierten Gerstenpflanzen werden in einem Gewächshaus bei ca. 22° C aufgestellt und der Pilzbefall nach 10 Tagen beurteilt.

b) Systemische Wirkung

Zu ca. 8 cm hohen Gerstenpflanzen wird eine aus Spritzpulver des Wirkstoffes hergestellte Spritzbrühe gegossen (0,006 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden werden die behandelten Pflanzen mit Konidien des Pilzes bestäubt. Die infizierten Gerstenpflanzen werden in einem Gewächshaus bei ca. 22° C aufgestellt und der Pilzbefall nach 10 Tagen beurteilt.

Verbindungen aus den Tabellen 1 - 3 besonders 1.009, 1.012, 1.065, 1.115, 1.127, 1.137, 3.077, 3.084 und 3.086 zeigen gute Wirksamkeit gegen Erysiphe-Pilze. Unbehandelte jedoch infizierte Kontrollpflanzen weisen dagegen einen Erysiphe-Befall von 100 % auf.

Beispiel 3.4: Residual-protektive Wirkung gegen Venturia inaequalis auf Apfeltrieben

Apfelstecklinge mit 10-20 cm langen Frischtrieben werden mit einer aus Spritzpulver des Wirkstoffes hergestellten Spritzbrühe (0,06 % Aktivsubstanz) besprüht. Nach 24 Stunden werden die behandelten Pflanzen mit einer Konidiensuspension des Pilzes infiziert. Die Pflanzen werden dann während 5 Tagen bei 90 - 100 % relative Luftfeuchtigkeit inkubiert und während 10 weiteren Tagen in einem Gewächshaus bei 20 - 24° C aufgestllt. Der Schorfbefall wird 15 Tage nach der Infektion beaurteilt. Verbindungen aus den Tabellen 1 - 3 zeigen gute Wirksamkeit gegen Venturia. Unbehandelte jedoch infizierte Triebe weisen dagegen einen 100%igen Venturia-Befall auf.

Beispiel 3.5: Wirkung gegen Botrytis cinerea auf Bohnen

Residual protektive Wirkung

Ca. 10 cm hohe Bohnen-Pflanzen werden mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach einer Inkubation der infizierten Pflanzen während 3 Tagen bei 95-100 % relativer Luftfeuchtigkeit und 21 °C erfolgt die Beurteilung des Pilzbefalls. Verbindungen aus den Tabellen 1 - 3 hemmen die Pilzinfektion sehr stark. Unbehandelte jedoch infizierte Kontrollpflanzen weisen dagegen einen Botrytis-Befall von 100 % auf. Sehr gut wirkten die Verbindungen 1.009, 1.012, 1.065, 1.115, 1.127, 1.137, 3.077, 3.084 und 3.086.

Beispiel 3.6: Wirkung gegen Pyricularia oryzae auf Reispflanzen

a) Residual-protektive Wirkung

Reispflanzen werden nach zweiwöchiger Anzucht mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach 5 Tagen Inkubation bei 95-100 % relativer Luftfeuchtigkeit und 24 °C wird der Pilzbefall beurteilt.

b) Systemische Wirkung

Zu zwei Wochen alten in für Blumen üblichen Tontöpfen wachsenden Reispflanzen wird eine aus Spritzpulver des Wirkstoffes hergestellte Spritzbrühe gegossen (0,006 % Aktivsubstanz bezogen auf das Erdvolumen). Darauf werden die Töpfe mit Wasser soweit gefüllt, dass die untersten Stengelteile der Reispflanzen im Wasser stehen. Nach 48 Stunden werden die behandelten Reispflanzen mit einer Konidiensuspension des Pilzes infiziert. Nach einer Inkubation der infizierten Pflanzen während 5 Tagen bei 95-100 % relativer Luftfeuchtigkeit und ca. 24° wird der Pilzbefall beurteilt.

Verbindungen aus den Tabellen zeigen gute Wirksamkeit gegen den Pyriculariapilz. Unbehandelte, jedoch infizierte Kontrollpflanzen weisen dagegen einen Pyricularia-Befall von 100 % auf. So hemmen die Verbindungen der Tabellen 1 - 3 insbesondere 1.115 und 3.040 den Pilzbefall bis auf 0 bis 5 %.

Beispiel 3.7: Wirkung gegen Tilletia caries an Weizen

Künstlich mit Brandsporen von Tilletia caries infizierte Wintergerste der Sorte Porbus (3 g trockenes Sporenmaterial auf 1 kg Saatgut) wird auf einer Mischrolle mit dem zu prüfenden Fungizid gebeizt, wobei eine Konzentration von 60 ppm AS (bezogen auf das Gewicht des Saatgutes) zur Anwendung gelangt.

Der infizierte und behandelte Weizen wird im Oktober im Freiland mittels einer Sämaschine auf Parzellen von 2 m Länge und 3 Saatreihen mit dreifacher Wiederholung ausgesät.

Zur Ermittlung der Wirkstoffaktivität werden zum Zeitpunkt der Aehrenreife der prozentuale Anteil Tilletia-befallener Aehren ausgezählt.

Verbindungen aus den Tabellen zeigen gute Wirksamkeit gegen Tilletia. Unbehandelte jedoch infizierte Kontrollpflanzen weisen dagegen einen Tilletia-Befall von 100 % auf. So hemmen die Verbindungen der Tabellen 1 - 3 den Pilzbefall bis auf 0 bis 5 %.

Beispiel 3.8: Wirkung gegen Helminthosporium gramineum an Gerste

Auf natürliche Weise mit Helminthosporium gramineum infizierte Wintergerste der Sorte "CI" wird auf einer Mischrolle mit dem zu prüfenden Fungizid gebeizt, wobei eine Konzentration von 60 ppm AS (bezogen auf das Gewicht des Saatgutes) zur Anwendung gelangt.

Die infizierte und behandelte Gerste wird im Oktober im Freiland mittels einer Sämaschine auf Parzellen von 2 m Länge und 3 Saatreihen mit dreifacher Wiederholung ausgesät.

Bis zur Befallsauswertung wird die Versuchspflanzung unter normalen Feldbedingungen kultiviert.

Zur Ermittlung der Wirkstoffaktivität werden zum Zeitpunkt des Aehrenschiebens der prozentuale Anteil Helminthosporium-befallener Halme ausgezählt.

Die Verbindungen der Tabellen 1 - 3 zeigen gute Wirksamkeit gegen Helminthosporium. Sie hemmen den Pilzbefall bis auf 0 - 5 % während unbehandelte infizierte Kontrollpflanzen einen Helminthosporium-Befall von 100 % aufweisen.

Beispiel 3.9: Wirkung gegen Phytophthora auf Tomatenpflanzen

a) Residual-protektive Wirkung

Tomatenplfanzen wurden nach 3-wöchiger Anzucht mit einer aus Spritzpulver des Wirkstoffes hergestellten Spritzbrühe (0,006 % Aktivsubstanz) besprüht. Nach 24 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100 % relativer Luftfeuchtigkeit und 20 °C.

b) Residual-kurative Wirkung

Tomatenpflanzen wurden nach 3-wöchiger Anzucht mit einer Sporangien suspension des Pilzes infiziert. Nach einer Inkubation von 22 Stunden in einer Feuchtkammer bei 90-100 % relativer Luftfeuchtigkeit und 20 °C wurden die infizierten pflanzen getrocknet und mit einer aus Spritzpulver des Wirkstoffes hergestellten Spritzbrühe (0,006 % Aktivsubstanz) besprüht. Nach dem Antrocknen des Spritzbelages wurden die behandelten Pflanzen wieder in die Feuchtkammer gebracht. Die Beurteilung des Pilzbefalls erfolgte 5 Tage nach der Infektion.

c) Systemische Wikrung

Zu Tomatenpflanzen wurde nach 3-wöchiger Anzucht eine aus Spritzpulver des Wirkstoffes hergestellte Spritzbrühe gegossen (0,002 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100 % relativer Luftfeuchtigkeit und 20 °C.

Verbindungen aus Tabellen 1 - 3 zeigen gegen Phytophthora gute Wirksamkeit. So reduzieren z.B. die Verbindungen 1.009, 1.043, 1.052, 1.115, 1.127 und 3.040 den Phytophthora-Befall bis auf 5-20 %. Unbehandelte aber infizierte Kontrollpflanzen weisen dagegen einen Phytophthora-Befall von 100 % auf.

Beispiel 3.10: Wirkung gegen Rhizoctonia solani (Bodenpilz auf Reispflanzen

a) Protektiv-lokale Bodenapplikation

12 Tage alte Reispflanzen werden mit einer aus einer Formulierung des Wirkstoffes hergestellten Spritzbrühe (0,006 % Aktivsubstanz), ohne oberirdische Pflanzenteile zu kontaminieren, angegossen. Zur Infizierung der behandelten Pflanzen wird eine Suspension von Myzel und Sklerotien von R. solani auf die Bodenoberfläche gegeben. Nach 6-tägiger Inkubation bei 27 °C (Tag), bzw. 23 °C (Nacht) und 100 % rel. Luftfeuchtigkeit (Feuchtkasten) in der Klimakkamer wird der Pilzbefall auf Blattscheide, Blättern und Stengel beurteilt.

b) Protektiv-lokale Blattapplikation

12 Tage alte Reispflanzen werden mit einer aus einer Formulierung der Wirkstoffe hergestellten Spritzbrühe besprüht. Ein Tag später werden die behandelten Pflanzen mit einer Suspension von Myzel und Sklerotien von R. solani infiziert. Nach 6-tägiger Inkubation bei 27 °C (Tag), bzw. 23 °C (Nacht) und 100 % rel. Luftfeuchtigkeit (Feuchtkasten) in der Klimakammer wird der Pilzbefall auf Blattscheide, Blättern und Stengel beurteilt.

Die geprüften Verbindungen der Tabellen 1 - 3 zeigten in diesem Versuch gute Wirkung. Am ausgeprägtesten war diejenige der Verbindung 3.040.

Beispiel 3.11: Wirkung gegen Xanthomonas oryzae auf Reis (Oryza sativa)

a) Residual-protektive Wirkung

Reispflanzen der Sorte "Calora" oder "S6" werden nach 3-wöchiger Anzucht im Gewächshaus mit der

Prüfsubstanz in Form einer Spritzbrühe (0,02 % Aktivsubstanz) besprüht. Nach eintägigem Antrocken dieses Spritzbelags werden die Pflanzen in einem Klimaraum bei 24 °C und 75-85 % relativer Luftfeuchtigkeit aufgestellt und infiziert. Die Infektion erfolgt, indem die Blattspitzen mit einer Schere, die zuvor in eine Suspension von Xanthomonas oryzae eingetaucht worden war, abgeschnitten werden. Nach 10-tägiger Inkubation werden die angeschnittenen Blätter bei Befall welk, rollen sich ein und werden nekrotisch. Das Ausmass dieser Krankheitssymptome dient zur Beurteilung der residual Wirksamkeit der Prüfsubstanz.

b) Systemische Wirkung

Reispflanzen der Sorte "Calora" oder "S6" werden nach 3-wöchiger Anzucht im Gewächshaus mit einer Suspension der Prüfsubstanz begossen (0,006 % Aktivsubstanz bezogen auf das Erdvolumen). Drei Tage nach dieser Behandlung werden die Pflanzen in einem Klimaraum bei 24 °C und 75-85 % relativer Luftfeuchtigkeit aufgestellt und infiziert. Die Infektion erfolgt, indem die Blattspitzen mit einer Schere, die zuvor in eine Suspension von Xanthomonas oryzae eingetaucht worden war, abgeschnitten werden. Nach 10-tägiger Inkubation werden die angeschnittenen Blätter bei Befall welk, rollen sich ein und werden nekrotisch. Das Ausmass dieser Krankheitssymptome dient zur Beurteilung der systemischen Wirksamkeit der Prüfsubstanz.

Die geprüften Verbindungen aus den Tablellen 1 und 3 zeigten eine gute Wirkung gegen Xanthomonas oryzae. So reduzierten z.b. im Test (a) und im Test (b) die Verbindungen 1.127 und 1.137 Pilzbefall auf 0 bis 20 %. Unbehandelte jedoch infizierte Kontrollpflanzen wiesen dagegen einen Krankheitsbefall von 100 % auf.

**Patentansprüche**

**Patentansprüche für folgende Vertragsstaaten : AT, BE, CH, DE, FR, GB, IT, LI, NL**

**1.** Die Verwendung von 2-Chlor-4-trifluormethyl-thiazol-5-carbonsäurederivaten der Formel Ia

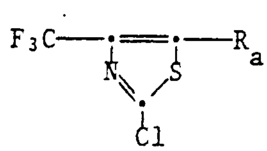

(Ia)

worin $R_a$ einen Rest Cyan, $-COXR_1$, $-CONR_3R_4$ oder -CO-D,

X Sauerstoff oder Schwefel,

$R_1$ Wasserstoff; $C_1$-$C_{18}$ Alkyl oder $C_3$-$C_{18}$ Cycloalkyl, welches unsubstituiert oder substituiert durch Halogen, eine Gruppe $-YR_2$, A, Nitro, $-N(R_3)COA$, $-[N(R_3)]_m-C(A)=NC_1-C_4$ Alkyl, $-[N(R_3)]_m-C(A)=NH$ oder $-[N(R_3)]_m-CO-N[(CO)_mR_3]N(R_3)$, $-[N(R_3)]_m-CON[(CO)_m-R_3]-N(R_3)_m-(CO)_mR_4$, in welchem Rest einer der Indices m Null sein muss, ferner kann der $C_1$-$C_{18}$ Alkyl oder $C_3$-$C_{18}$ Cycloalkylrest substituiert sein durch Cyano, einen Rest $-C(X)_m-XR_{10}$, $-XCXR_{10}$, $-(X)_m-CXA$, $-(X)CXN(R_3)N(R_3)R_4$, $-CHA-COOR_{10}$, $-C(OR_7)(OR_8)R_9$, $PO(R_5)R_6$, $C_3$-$C_8$-Cycloalkyl oder $C_5$-$C_8$ Cycloalkenyl; $R_1$ ist ferner $C_3$-$C_8$ Alkenyl oder $C_3$-$C_{18}$ Cycloalkenyl, welches unsubstituiert oder substituiert ist durch Halogen, $C_1$-$C_4$ Alkoxy, $C_1$-$C_4$ Alkylthio, $C_1$-$C_4$ Halogenalkoxy, $C_1$-$C_4$ Halogenalkyl oder einen Rest $-CO(O)_m-R_{10}$, -COA, $-CON(A)R_3$ oder $PO(R_5)R_6$-, $R_1$ ist ferner $C_3$-$C_8$ Alkinyl, welches unsubstituiert ist oder ein Rest $-(E)_mU$ oder $-(E)_m-Q$,

$R_2$ $C_1$-$C_8$Alkyl oder $C_3$-$C_8$ Cycloalkyl, welches unsubstituiert oder durch $C_1$-$C_8$Alkoxy, $C_1$-$C_8$ Alkylthio, $C_3$-$C_8$ Alkoxyalkoxy, Halogen, Cyan oder einen Rest $-CX(X)_mR_{10}$, $-(X)_m-CX-A$, $-(X)_mCXR_{10}$, $N(R_3)COA$, $-[(N(R_3)]_m-C(A)=NH$, $-[N(R_3)]_m-C(A)=NC_1-C_4$Alkyl, A, -X-U oder -XQ substituiert ist, $R_2$ bedeutet ferner $C_3$-$C_8$Alkenyl oder $C_3$-$C_8$ Cycloalkenyl, welches unsubstituiert oder durch Halogen substituiert ist, oder einen Rest $-(E)_mU$ oder $-(E)_mQ$;

m ist Null oder 1;

Y ist Sauerstoff, Schwefel, SO oder $SO_2$,

A ist ein Rest $-N(R_3)R_4$,

D ist ein Rest $-N(R_3)N(R_4)(CO)_mR_3$;

$R_3$ und $R_4$ sind unabhängig voneinander je Wasserstoff, $C_1$-$C_8$Alkyl oder $C_3$-$C_8$Cycloalkyl, welches unsubstituiert oder durch $C_1$-$C_8$Alkoxy, $C_2$-$C_8$Alkoxyalkoxy, $C_1$-$C_8$Alkylthio, Cyano, einen Rest $-COOR_{10}$, $C_1$-$C_4$Alkylcarbamoyl, Di-$C_1$-$C_4$alkylcarbamoyl, $C_1$-$C_4$Alkylamino, Di-$C_1$-$C_4$Alkylamino, Piperidinocarbonyl, Pyrrolidinocarbonyl, Piperidino oder Pyrrolidino substituiert ist; $R_3$ und $R_4$ sind ferner $C_3$-

$C_8$Alkenyl oder $C_3$-$C_8$Cycloalkenyl, welches unsubstituiert oder substituiert ist durch Halogen, $C_1$-$C_8$ Alkoxy, $C_3$-$C_8$Cycloalkenyl, oder einen Rest Cyan, -$COOR_{10}$, $C_1$-$C_4$Alkylcarbamoyl oder Piperidinocarbamoyl substituiert ist, $R_3$ und $R_4$ sind ferner $C_3$-$C_8$Alkinyl, welches unsubstituiert oder durch U substituiert ist, oder $R_3$ und $R_4$ bedeuten einen Rest -$(E)_m$-U oder -$(E)_m$-Q;

$R_3$ und $R_4$ zusammen mit dem Stickstoffatom, an das sie gebunden sind, bilden auch einen gesättigten oder ungesättigten 5 - 9 gliedrigen Heterocyclus, der ein- oder mehrfach durch Sauerstoff, Schwefel, Stickstoff, -NH-, -$NC_1$-$C_4$Alkyl, -CO- oder -$C(OR_7)OR_8$- unterbrochen und durch Halogen, Cyan, $C_1$-$C_8$Alkoxy, Amino, $C_1$-$C_4$Alkylamino, Di-$C_1$-$C_4$-Alkylamino, oder einen Rest -$COOR_{10}$ substituiert sein kann;

$R_5$ und $R_6$ unabhängig voneinander je Hydroxy, $C_1$-$C_4$Alkyl oder $C_2$-$C_4$Alkoxy;

$R_7$ und $R_8$ sind unabhängig voneinander je $C_1$-$C_4$Alkyl oder

$R_7$ und $R_8$ bilden zusammen eine 2 - 4 gliedrige Alkylenkette,

$R_9$ und $R_{10}$ sind unabhängig voneinander je Wasserstoff, $C_1$-$C_3$Alkyl, $C_3$-$C_8$Cycloalkyl, $C_3$-$C_8$Alkenyl, $C_3$-$C_8$Cycloalkenyl, $C_3$-$C_8$Alkinyl, $C_2$-$C_8$Alkoxyalkyl, $C_4$-$C_8$Alkoxyalkoxyalkyl, $C_1$-$C_4$Halogenalkyl-$(C_1$-$C_3$-$Alkyl)_m$U, -$(C_1$-$C_3$$Alkyl)_m$Q, $C_1$-$C_4$Halogenalkoxy, $C_1$-$C_4$ Halogenalkoxy-$C_1$-$C_4$alkyl;

U ist ein Phenyl- oder Naphthylrest, der unsubstituiert oder ein-oder mehrfach durch Halogen, $C_1$-$C_4$Alkyl, -Y-$C_1$-$C_4$Alkyl, $C_1$-$C_4$-Halogenalkyl, $C_1$-$C_4$Halogenalkoxy, Cyano, Nitro, -COOH, -$COOR_7$, -$CONH_2$, -$CONHR_7$, -$CON(R_7)_2$, $SO_2NHR_7$, $SO_2N(R_7)_2$ Pyrrolidino, Piperidino, Pyrrolidinocarbonyl oder Piperidinocarbonyl substituiert ist;

E ist eine $C_1$-$C_8$Alkylen, $C_2$-$C_8$Alkenylenkette, die unsubstituiert oder durch Halogen, $C_1$-$C_4$Alkoxy, $C_1$-$C_4$Alkylthio, $C_1$-$C_4$ Halogenalkoxy oder einen Rest -$CO(O)_mR_{10}$, -$(CO)_m$-A, -$(CO)_m$Q substituiert und/oder durch ein Glied -CO- oder -$C(OR_7)OR_8$- unterbrochen ist,

Q ist ein gesättigter oder ungesättigter 5 - 12 gliedriger Heterocyclus, der 1 - 4 Heteroatome oder eine Sulfinyl- oder Sulfonylgruppe auch in Kombination mit 1 - 2 Heteroatomen enthalten kann, der durch eine oder zwei Carbonylgruppen unterbrochen und benzanneliert sein kann,

zur Bekämpfung oder Verhütung eines Befalles von Kulturpflanzen durch pathogene Mikroorganismen.

**2.** Die Verwendung gemäss Anspruch 1 von 2-Chlor-4-trifluormethyl-thiazol-5-carbonsäurederivaten der Formel Ib

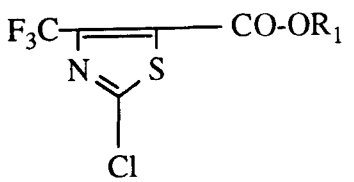

(Ib)

worin $R_1$ die im Anspruch 1 gegebene Bedeutung hat.

**3.** 2-Chlor-4-trifluormethyl-thiazol-5-carbonsäurederivaten der Formel Ib gemäss Anspruch 2, worin $R_1$ den

Tetrahydropyran-2-ylmethyl-
2,2-Dimethyl-1,3-dioxolan-4-ylmethyl-
1,2-Dihydrobenz-1,4-dioxan-2-ylmethyl-
Thiophen-2-ylmethyl-
3,4 Methylendioxybenzyl-
5-Methylthiazol-4-ylethyl-
para Tolyl-eth-1-yl
Bornyl-
Norbornyl-
Fenchyl-
Menthyl-
Cyanoethyl-
Phenoxyethyl-
Methylsulfonylethyl-
Phenylthioethyl-
Ethoxycarbonylmethyl-
Ethoxycarbonyleth-1-yl-

5,5-Dimethyl-tetrahydrofuran-2-on-3-yl-
2-Oxopyrrolinomethyl-
α-Methoxycarbonyl-benzyl-
α-Cyano-benzyl-
α-Benzoyl-benzyl- oder
α-Methoxycarbonyl-α-phenyl-benzyl- oder den Morpholinomethylrest bedeutet.

**4.** 2-Chlor-4-trifluormethyl-thiazol-5-carbonsäurederivate der Formel Ib gemäss Anspruch 2, worin $R_1$ einen $C_1$-$C_{12}$-Alkylrest bedeutet, der durch $-(X)_m$-CX-A substituiert ist, während A, m und X die im Anspruch 1 gegebene Bedeutung haben.

**5.** 2-Chlor-4-trifluormethyl-thiazol-5-carbonsäurederivate der Formel Ib gemäss Anspruch 4, worin m Null und X Sauerstoff bedeutet, während A die im Anspruch 1 gegebene Bedeutung hat.

**6.** 2-Chlor-4-trifluormethyl-thiazol-5-carbonsäurederivate der Formel Ib gemäss Anspruch 2, worin $R_1$ den Azepinomethyl-, N-(2,6-Dimethylphenyl)-N-(methoxycarbonyleth-1-yl)-carbamoylmethyl- oder den 2-Piperidino-eth-1-ylrest bedeutet.

**7.** Die Verwendung gemäss Anspruch 1 der 2-Chlor-4-trifluormethyl-thiazol-5-carbonsäurederivate der Formel Ic,

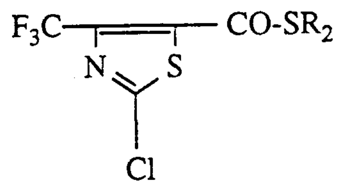

(Ic)

worin $R_2$ die im Anspruch 1 gegebene Bedeutung hat, zur Bekämpfung oder Verhütung eines Befalles von Kulturpflanzen durch pathogene Mikroorganismen.

**8.** 2-Chlor-4-trifluormethyl-thiazol-5-carbonsäurederivate der Formel Id,

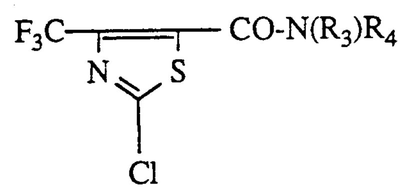

worin eines von $R_3$ und $R_4$ Wasserstoff, $C_1$-$C_8$-Alkyl unsubstituiert, das andere oder beide $C_3$-$C_8$-Cycloalkyl, welches unsubstituiert ist, oder $C_1$-$C_8$-Alkyl oder $C_3$-$C_8$Cycloalkyl, welches durch $C_1$-$C_8$-Alkoxy, $C_2$-$C_8$Alkoxyalkoxy, $C_1$-$C_8$Alkylthio, Cyano, einen Rest $-COOR_{10}$, $C_1$-$C_4$Alkylcarbamoyl, Di-$C_1$-$C_4$alkylcarbamoyl, $C_1$-$C_4$Alkylamino, Di-$C_1$-$C_4$Alkylamino, Piperidinocarbonyl, Pyrrolidinocarbonyl, Piperidino oder Pyrrolidino substituiert ist; $R_3$ und $R_4$ sind ferner $C_3$-$C_8$Alkenyl oder $C_3$-$C_8$Cycloalkenyl, welches unsubstituiert oder substituiert ist durch Halogen, $C_1$-$C_8$ Alkoxy, $C_3$-$C_8$Cycloalkenyl, oder einen Rest Cyan, $-COOR_{10}$, $C_1$-$C_4$Alkylcarbamoyl, Di-$C_1$-$C_4$alkylcarbamoyl, U, Pyrrolidino-carbamoyl oder Piperidinocarbamoyl substituiert ist, $R_3$ und $R_4$ sind ferner $C_3$-$C_8$Alkinyl, welches unsubstituiert oder durch U substituiert ist, oder $R_3$ und $R_4$ bedeuten einen Rest $-(E)_m$-U oder $-(E)_m$-Q, sind, und
$R_3$ und $R_4$ zusammen mit dem Stickstoffatom, an das sie gebunden sind, bilden auch einen gesättigten oder ungesättigten 5 - 9 gliedrigen Heterocyclus, der ein- oder mehrfach durch Sauerstoff, Schwefel, Stickstoff, -NH-, -N$C_1$-$C_4$Alkyl, -CO- oder $-C(OR_7)OR_8$- unterbrochen und durch Halogen, Cyan, $C_1$-$C_8$Alkoxy, Amino, $C_1$-$C_4$Alkylamino, Di-$C_1$-$C_4$-Alkylamino, oder einen Rest $-COOR_{10}$ substituiert sein kann.

**9.** Fungizides Mittel, enthaltend als Wirkstoff ein 2-Chlor-4-trifluormethyl-thiazol-5-carbonsäure derivat der

Formel Id

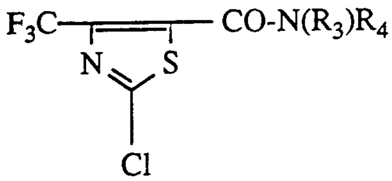

(Id)

worin $R_3$ und $R_4$ die im Anspruch 8 gegebene Bedeutung haben.

**10.** Die Verwendung gemäss Anspruch 1 von 2-Chlor-trifluormethyl-thiazol-5-carbonsäurederivaten der Formel Id

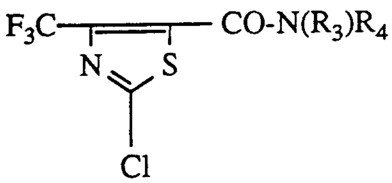

(Id)

worin $R_3$ und $R_4$ die im Anspruch 8 gegebene Bedeutung haben, zur Bekämpfung oder Verhütung eines Befalles von Kulturpflanzen durch pathogene Mikroorganismen.

**11.** 2-Chlor-4-trifluormethyl-thiazol-5-carbonsäurehydrazide gemäss Anspruch 1 der Formel Ie

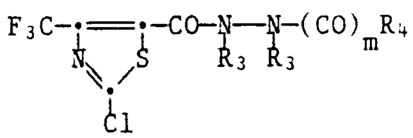

(Ie)

worin m, die $R_3$ und $R_4$ unabhängig voneinander eine im Anspruch 8 gegebene Bedeutung haben.

**12.** 2-Chlor-4-trifluormethyl-thiazol-5-carbonsäurehydrazide gemäss Anspruch 1 der Formel If

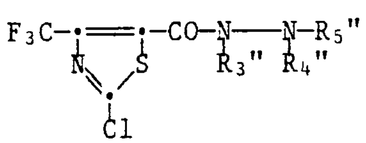

(If)

worin $R_3$", $R_4$" und $R_5$" unabhängig voneinander je Wasserstoff, $C_1$-$C_8$-Alkyl-$C_3$-$C_8$-Cycloalkyl, Phenyl oder Benzyl bedeuten wobei Phenyl und Benzyl unsubstituiert oder durch Halogen, $C_1$-$C_4$-Alkyl, $C_1$-$C_4$-Haloalkyl, $C_1$-$C_4$-Alkoxy, $C_1$-$C_4$-Haloalkyl, Nitro, Carboxyl, $C_1$-$C_4$-Alkoxycarbonyl, Carbamoyl, Methylcarbamoyl substituiert sind, $R_3$" und $R_4$" zusammen auch eine 4-5 gliedrige Alkylenkette bilden, die durch Sauerstoff, Schwefel, Imino oder $C_1$-$C_4$-Alkylimino unterbrochen und durch $C_1$-$C_4$-Alkyl ein- oder mehrmals substituiert sein kann, bedeuten.

**13.** 2-Chlor-4-trifluormethyl-thiazol-carbonsäureamide der Formel Id gemäss Anspruch 1, worin -N($R_3$)$R_4$ den
-N-2,4-Dichlorbenzyl-N-methylcarbamoyl-,
-N-Benzyl-N-isopropylcarbamoyl-,
-N-Cyclohexyl-N-methoxycarbamoylethyl-carbamoyl-,

-N-2,6-Dimethylphenyl-N-methoxycarbamoyleth-1-yl-carbamoyl oder den -N-(1-Cyanopent-1-yl)-N-methyl-carbamoylrest bedeutet.

**14.** Verfahren zur Bekämpfung oder Verhütung eines Befalls von Kulturpflanzen durch phytopathogene Mikroorganismen, dadurch gekennzeichnet, dass man eine gemäss Anspruch 1 definierte Verbindung der Formel Ia auf die Pflanze oder deren Standort appliziert.

**15.** Verwendung von Verbindungen der Formel I gemäss Anspruch 1 oder sie enthaltender Mittel zur Bekämpfung und/oder zur präventiven Verhütung eines Befalls von Mikroorganismen.

**16.** Verwendung gemäss Anspruch 15, dadurch gekennzeichnet, dass es sich bei den Mikroorganismen um phytopathogene Pilze handelt.

**17.** Verfahren gemäss Anspruch 14, dadurch gekennzeichnet, dass die Pflanzenteile das Saatgut sind.

**18.** Verfahren gemäss Anspruch 14, dadurch gekennzeichnet, dass Reispflanzen behandelt werden.

**19.** Verwendung gemäss Anspruch 15, dadurch gekennzeichnet, dass es sich bei den Mikroorganismen um bodenbürtige phytopathogene Bakterien handelt.

**Patentansprüche für folgenden Vertragsstaat : ES**

**1.** Die Verwendung von 2-Chlor-4-trifluormethyl-thiazol-5-carbonsäurederivaten der Formel Ia

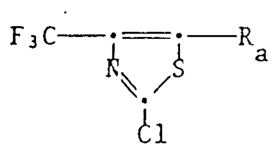

(Ia)

worin $R_a$ einen Rest Cyan, $-COXR_1$, $-CONR_3R_4$ oder -CO-D,
X Sauerstoff oder Schwefel,
$R_1$ Wasserstoff; $C_1$-$C_{18}$ Alkyl oder $C_3$-$C_{18}$ Cycloalkyl, welches unsubstituiert oder substituiert durch Halogen, eine Gruppe $-YR_2$, A, Nitro, $-N(R_3)COA$, $-[N(R_3)]_m-C(A)=NC_1-C_4$ Alkyl, $-[N(R_3)]_m-C(A)=NH$ oder $-[N(R_3)]_m-CO-N[(CO)_mR_3]N(R_3)$, $-[N(R_3)]_m-CON[(CO)_m-R_3]N(R_3)_m-(CO)_mR_4$, in welchem Rest einer der Indices m Null sein muss, ferner kann der $C_1$-$C_{18}$ Alkyl oder $C_3$-$C_{18}$ Cycloalkylrest substituiert sein durch Cyano, einen Rest $-C(X)_m-XR_{10}$, $-XCXR_{10}$, $-(X)_m-CXA$, $-(X)CXN(R_3)N(R_3)R_4$, $-CHA-COOR_{10}$, $-C(OR_7)(OR_8)R_9$, $PO(R_5)R_6$, $C_3$-$C_8$-Cycloalkyl oder $C_5$-$C_8$ Cycloalkenyl; $R_1$ ist ferner $C_3$-$C_8$ Alkenyl oder $C_3$-$C_{18}$ Cycloalkenyl, welches unsubstituiert oder substituiert ist durch Halogen, $C_1$-$C_4$ Alkoxy, $C_1$-$C_4$ Alkylthio, $C_1$-$C_4$ Halogenalkoxy, $C_1$-$C_4$ Halogenalkyl oder einen Rest $-CO(O)_m-R_{10}$, -COA, $-CON(A)R_3$ oder $PO(R_5)R_6$-, $R_1$ ist ferner $C_3$-$C_8$ Alkinyl, welches unsubstituiert ist oder ein Rest $-(E)_mU$ oder $-(E)_m$-Q,
$R_2$ $C_1$-$C_8$Alkyl oder $C_3$-$C_8$ Cycloalkyl, welches unsubstituiert oder durch $C_1$-$C_8$Alkoxy, $C_1$-$C_8$ Alkylthio, $C_3$-$C_8$ Alkoxyalkoxy, Halogen, Cyan oder einen Rest $-CX(X)_mR_{10}$, $-(X)_m$-CX-A, $-(X)_mCXR_{10}$, $N(R_3)COA$, $-[N(R_3)]_m-C(A)=NH$, $-[N(R_3)]_m-C(A)=NC_1-C_4$Alkyl, A, -X-U oder -XQ substituiert ist, $R_2$ bedeutet ferner $C_3$-$C_8$Alkenyl oder $C_3$-$C_8$ Cycloalkenyl, welches unsubstituiert oder durch Halogen substituiert ist, oder einen Rest $-(E)_mU$ oder $-(E)_mQ$;
m ist Null oder 1;
Y ist Sauerstoff, Schwefel, SO oder $SO_2$,
A ist ein Rest $-N(R_3)R_4$,
D ist ein Rest $-N(R_3)N(R_4)(CO)_mR_3$;
$R_3$ und $R_4$ sind unabhängig voneinander je Wasserstoff, $C_1$-$C_8$Alkyl oder $C_3$-$C_8$Cycloalkyl, welches unsubstituiert oder durch $C_1$-$C_8$Alkoxy, $C_2$-$C_8$Alkoxyalkoxy, $C_1$-$C_8$Alkylthio, Cyano, einen Rest $-COOR_{10}$, $C_1$-$C_4$Alkylcarbamoyl, Di-$C_1$-$C_4$alkylcarbamoyl, $C_1$-$C_4$Alkylamino, Di-$C_1$-$C_4$Alkylamino, Piperidinocarbonyl, Pyrrolidinocarbonyl, Piperidino oder Pyrrolidino substituiert ist; $R_3$ und $R_4$ sind ferner $C_3$-$C_8$Alkenyl oder $C_3$-$C_8$Cycloalkenyl, welches unsubstituiert oder substituiert ist durch Halogen, $C_1$-$C_8$ Alkoxy, $C_3$-$C_8$Cycloalkenyl, oder einen Rest Cyan, $-COOR_{10}$, $C_1$-$C_4$Alkylcarbamoyl oder Piperidinocar-

bamoyl substituiert ist, $R_3$ und $R_4$ sind ferner $C_3$-$C_8$Alkinyl, welches unsubstituiert oder durch U substituiert ist, oder $R_3$ und $R_4$ bedeuten einen Rest -$(E)_m$-U oder -$(E)_m$-Q;
$R_3$ und $R_4$ zusammen mit dem Stickstoffatom, an das sie gebunden sind, bilden auch einen gesättigten oder ungesättigten 5 - 9 gliedrigen Heterocyclus, der ein- oder mehrfach durch Sauerstoff, Schwefel, Stickstoff, -NH-, -$NC_1$-$C_4$Alkyl, -CO- oder -$C(OR_7)OR_8$- unterbrochen und durch Halogen, Cyan, $C_1$-$C_8$Alkoxy, Amino, $C_1$-$C_4$Alkylamino, Di-$C_1$-$C_4$-Alkylamino, oder einen Rest -$COOR_{10}$ substituiert sein kann;
$R_5$ und $R_6$ unabhängig voneinander je Hydroxy, $C_1$-$C_4$Alkyl oder $C_2$-$C_4$Alkoxy;
$R_7$ und $R_8$ sind unabhängig voneinander je $C_1$-$C_4$Alkyl oder
$R_7$ und $R_8$ bilden Zusammen eine 2 - 4 gliedrige Alkylenkette,
$R_9$ und $R_{10}$ sind unabhängig voneinander je Wasserstoff, $C_1$-$C_3$Alkyl, $C_3$-$C_8$Cycloalkyl, $C_3$-$C_8$Alkenyl, $C_3$-$C_8$Cycloalkenyl, $C_3$-$C_8$Alkinyl, $C_2$-$C_8$Alkoxyalkyl, $C_4$-$C_8$Alkoxyalkoxyalkyl, $C_1$-$C_4$Halogenalkyl-($C_1$-$C_3$-Alkyl)$_m$U, -($C_1$-$C_3$Alkyl)$_m$Q, $C_1$-$C_4$Halogenalkoxy, $C_1$-$C_4$ Halogenalkoxy-$C_1$-$C_4$alkyl;
U ist ein Phenyl- oder Naphthylrest, der unsubstituiert oder ein-oder mehrfach durch Halogen, $C_1$-$C_4$Alkyl, -Y-$C_1$-$C_4$Alkyl, $C_1$-$C_4$Halogenalkyl, $C_1$-$C_4$Halogenalkoxy, Cyano, Nitro, -COOH, -$COOR_7$, -$CONH_2$, -$CONHR_7$, -$CON(R_7)_2$, $SO_2NHR_7$, $SO_2N(R_7)_2$ Pyrrolidino, Piperidino, Pyrrolidinocarbonyl oder Piperidinocarbonyl substituiert ist;
E ist eine $C_1$-$C_8$Alkylen, $C_2$-$C_8$Alkenylenkette, die unsubstituiert oder durch Halogen, $C_1$-$C_4$Alkoxy, $C_1$-$C_4$Alkylthio, $C_1$-$C_4$ Halogenalkoxy oder einen Rest -$CO(O)_mR_{10}$, -$(CO)_m$-A, -$(CO)_m$Q substituiert und/oder durch ein Glied -CO- oder -$C(OR_7)OR_8$- unterbrochen ist,
Q ist ein gesättigter oder ungesättigter 5 - 12 gliedriger Heterocyclus, der 1 - 4 Heteroatome oder eine Sulfinyl- oder Sulfonylgruppe auch in Kombination mit 1 - 2 Heteroatomen enthalten kann, der durch eine oder zwei Carbonylgruppen unterbrochen und benzanneliert sein kann,
zur Bekämpfung oder Verhütung eines Befalles von Kulturpflanzen durch pathogene Mikroorganismen.

2. Die Verwendung gemäss Anspruch 1 von 2-Chlor-4-trifluromethyl-thiazol-5-carbonsäurederivaten der Formel Ib

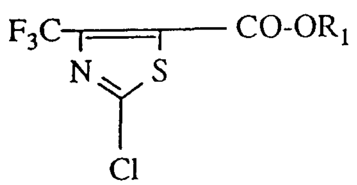

(Ib)

worin $R_1$ die im Anspruch 1 gegebene Bedeutung hat.

3. Die Verwendung gemäß Anspruch 1 von 2-Chlor-4-trifluormethyl-thiazol-5-carbonsäurederivaten der Formel Ib gemäss Anspruch 2, worin $R_1$ den
Tetrahydropyran-2-ylmethyl-
2,2-Dimethyl-1,3-dioxolan-4-ylmethyl-
1,2-Dihydrobenz-1,4,dioxan-2-ylmethyl-
Thiophen-2-ylmethyl-
3,4 Methylendioxybenzyl-
5-Methylthiazol-4-ylethyl-
para Tolyl-eth-1-yl
Bornyl-
Norbornyl-
Fenchyl-
Menthyl-
Cyanoethyl-
Phenoxyethyl-
Methylsulfonylethyl-
Phenylthioethyl-
Ethoxycarbonylmethyl-
Ethoxycarbonyleth-1-yl-
5,5-Dimethyl-tetrahydrofuran-2-on-3-yl-

2-Oxopyrrolinomethyl-
α-Methoxycarbonyl-benzyl-
α-Cyano-benzyl-
α-Benzoyl-benzyl- oder
α-Methoxycarbonyl-α-phenyl-benzyl- oder den Morpholinomethylrest bedeutet.

4. Die Verwendung gemäß Anspruch 1 von 2-Chlor-4-trifluormethyl-thiazol-5-carbonsäurederivate der Formel Ib gemäss Anspruch 2, worin $R_1$ einen $C_1$-$C_{12}$-Alkylrest bedeutet, der durch $-(X)_m$-CX-A substituiert ist, während A, m und X die im Anspruch 1 gegebene Bedeutung haben.

5. Die Verwendung gemäß Anspruch 1 von 2-Chlor-4-trifluormethyl-thiazol-5-carbonsäurederivate der Formel Ib gemäss Anspruch 4, worin m Null und X Sauerstoff bedeutet, während A die im Anspruch 1 gegebene Bedeutung hat.

6. Die Verwendung gemäß Anspruch 1 von 2-Chlor-4-trifluormethyl-thiazol-5-carbonsäurederivate der Formel Ib gemäss Anspruch 2, worin $R_1$ den Azepinomethyl-, N-(2,6-Dimethylphenyl)-N-(methoxycarb onyleth-1-yl)-carbamoylmethyl- oder den 2-Piperidino-eth-1-ylrest bedeutet.

7. Die Verwendung gemäss Anspruch 1 der 2-Chlor-4-trifluormethyl-thiazol-5-carbonsäurederivate der Formel Ic,

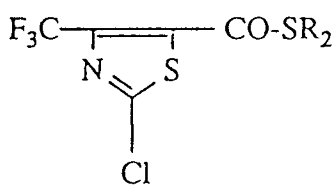

(Ic)

worin $R_2$ die im Anspruch 1 gegebene Bedeutung hat, zur Bekämpfung oder Verhütung eines Befalles von Kulturpflanzen durch pathogene Mikroorganismen.

8. Fungizides Mittel, enthaltend als Wirkstoff ein 2-Chlor-4-trifluormethyl-thiazol-5-carbonsäurederivat der Formel Id

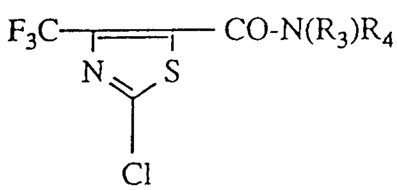

(Id)

worin eines von $R_3$ und $R_4$ Wasserstoff, $C_1$-$C_8$-Alkyl unsubstituiert, das andere oder beide $C_3$-$C_8$-Cycloalkyl, welches unsubstituiert ist, oder $C_1$-$C_8$-Alkyl oder $C_3$-$C_8$ Cycloalkyl, welches durch $C_1$-$C_8$-Alkoxy, $C_2$-$C_8$ Alkoxyalkoxy, $C_1$-$C_8$ Alkylthio, Cyano, einen Rest $-COOR_{10}$, $C_1$-$C_4$ Alkylcarbamoyl, Di-$C_1$-$C_4$ alkylcarbamoyl, $C_1$-$C_4$ Alkylamino, Di-$C_1$-$C_4$ Alkylamino, Piperidinocarbonyl, Pyrrolidinocarbonyl, Piperidino oder Pyrrolidino substituiert ist; $R_3$ und $R_4$ sind ferner $C_3$-$C_8$ Alkenyl oder $C_3$-$C_8$ Cycloalkenyl, welches unsubstituiert oder substituiert ist durch Halogen, $C_1$-$C_8$ Alkoxy, $C_3$-$C_8$ Cycloalkenyl, oder einen Rest Cyan, $-COOR_{10}$, $C_1$-$C_4$ Alkylcarbamoyl, Di-$C_1$-$C_4$ alkylcarbamoyl, U, Pyrrolidino-carbamoyl oder Piperidinocarbamoyl substituiert ist, $R_3$ und $R_4$ sind ferner $C_3$-$C_8$ Alkinyl, welches unsubstituiert oder durch U substituiert ist, oder $R_3$ und $R_4$ bedeuten einen Rest $-(E)_m$-U oder $-(E)_m$-Q, sind, und
$R_3$ und $R_4$ zusammen mit dem Stickstoffatom, an das sie gebunden sind, bilden auch einen gesättigten oder ungesättigten 5 - 9 gliedrigen Heterocyclus, der ein- oder mehrfach durch Sauerstoff, Schwefel, Stickstoff, -NH-, $-NC_1$-$C_4$ Alkyl, -CO- oder $-C(OR_7)OR_8$- unterbrochen und durch Halogen, Cyan, $C_1$-$C_8$ Alkoxy, Amino, $C_1$-$C_4$ Alkylamino, Di-$C_1$-$C_4$-Alkylamino, oder einen Rest $-COOR_{10}$ substituiert sein kann.

**9.** Die Verwendung gemäss Anspruch 1 von 2-Chlor-4-trifluormethyl-thiazol-5-carbonsäurederivaten der Formel Id

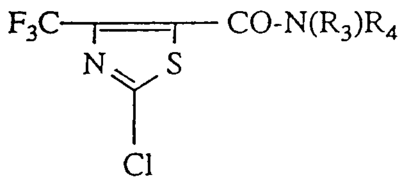

(Id)

worin $R_3$ und $R_4$ die im Anspruch 8 gegebene Bedeutung haben, zur Bekämpfung oder Verhütung eines Befalles von Kulturpflanzen durch pathogene Mikroorganismen.

**10.** Verfahren zur Bekämpfung oder Verhütung eines Befalls von kulturpflanzen durch phytopathogene Mikroorganismen, dadurch gekennzeichnet, dass man eine gemäss Anspruch 1 definierte Verbindung der Formel Ia auf die Pflanze oder deren Standort appliziert.

**11.** Verwendung von Verbindungen der Formel I gemäss Anspruch 1 oder sie enthaltender Mittel zur Bekämpfung und/oder zur präventiven Verhütung eines Befalls von Mikroorganismen.

**12.** Verwendung gemäss Anspruch 11, dadurch gekennzeichnet, dass es sich bei den Mikroorganismen um phytopathogene Pilze handelt.

**13.** Verfahren gemäss Anspruch 10, dadurch gekennzeichnet, dass die Pflanzenteile das Saatgut sind.

**14.** Verfahren gemäss Anspruch 10, dadurch gekennzeichnet, dass Reispflanzen behandelt werden.

**15.** Verwendung gemäss Anspruch 11, dadurch gekennzeichnet, dass es sich bei den Mikroorganismen um bodenbürtige phytopathogene Bakterien handelt.

**Claims**
**Claims for the following Contracting States : AT, BE, CH, DE, FR, GB, IT, LI, NL,**

**1.** The use of a 2-chloro-4-trifluoromethylthiazole-5-carboxylic acid derivative of formula Ia

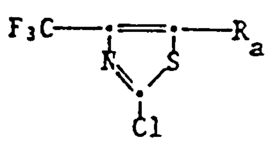

**(Ia),**

wherein $R_a$ is a cyano, $-COXR_1$, $-CONR_3R_4$ or -CO-D radical, X is oxygen or sulfur, $R_1$ is hydrogen, or $C_1$-$C_{18}$alkyl or $C_3$-$C_{18}$cycloalkyl which is unsubstituted or substituted by halogen, a group $-YR_2$, A, nitro, $-N(R_3)COA$, $-[N(R_3)]_m-C(A)=NC_1-C_4$alkyl, $-[N(R_3)]_m-C(A)=NH$, $-[N(R_3)]_m-CO-N\{(CO)_mR_3\}-N(R_3)$ or $-[N(R_3)]_m-CON[(CO)_m-R_3]-N(R_3)_m-(CO_m)R_4$, in which groups one of the indices m must be zero, furthermore the $C_1$-$C_{18}$alkyl or $C_3$-$C_{18}$cycloalkyl radical can be substituted by cyano or by a radical $-C(X)_m-XR_{10}$, $-XCXR_{10}$, $-(X)_m-CXA$, $-(X)CXN(R_3)N(R_3)R_4$, $-CHA-COOR_{10}$, $-C(OR_7)(OR_8)R_9$, $PO(R_5)R_6$, $C_3$-$C_8$cycloalkyl or $C_5$-$C_8$cycloalkenyl; $R_1$ further represents $C_3$-$C_8$alkenyl or $C_3$-$C_{18}$cycloalkenyl which is unsubstituted or substituted by halogen, $C_1$-$C_4$alkoxy, $C_1$-$C_4$alkylthio, $C_1$-$C_4$haloalkoxy, $C_1$-$C_4$haloalkyl or by a radical $-CO(O)_m-R_{10}$, -COA, $-CON(A)R_3$ or $PO(R_5)R_6$-; $R_1$ further represents $C_3$-$C_8$alkynyl which is unsubstituted or a radical $-(E)_mU$ or $-(E)_mQ$, $R_2$ represents $C_1$-$C_8$alkyl or $C_3$-$C_8$cycloalkyl which is unsubstituted or substituted by $C_1$-$C_8$alkoxy, $C_1$-$C_8$alkylthio, $C_3$-$C_8$alkoxyalkoxy, halogen, cyano or by a radical $-CX(X)_mR_{10}$, $-(X)_m-CX-A$, $-(X)_mCXR_{10}$, $N(R_3)COA$, $-[N(R_3)]_m-C(A)=NH$, $-[N(R_3)]_m-C(A)=NC_1$-$C_4$alkyl, A, -X-U or -XQ; $R_2$ further represents $C_3$-$C_8$alkenyl or $C_3$-$C_8$cycloalkenyl which is unsubstituted or substituted by halogen, or represents a radical $-(E)_mU$ or $-(E)_mQ$; m is zero or one; Y is oxygen, sulfur, SO or $SO_2$; A is a radical $-N(R_3)R_4$; D is a radical $-N(R_3)N(R_4)(CO)_mR_3$; $R_3$ and $R_4$ indepen-

dently of each other represent hydrogen, or $C_1$-$C_8$alkyl or $C_3$-$C_8$cycloalkyl which is unsubstituted or substituted by $C_1$-$C_8$alkoxy, $C_2$-$C_8$alkoxyalkoxy, $C_1$-$C_8$alkylthio, cyano, a radical -$COOR_{10}$, $C_1$-$C_4$alkylcarbamoyl, di-$C_1$-$C_4$alkylcarbamoyl, $C_1$-$C_4$alkylamino, di-$C_1$-$C_4$alkylamino, piperidinocarbonyl, pyrrolidinocarbonyl, piperidino or pyrrolidino; $R_3$ and $R_4$ further represent $C_3$-$C_8$alkenyl or $C_3$-$C_8$cycloalkenyl which is unsubstituted or substituted by halogen, $C_1$-$C_8$alkoxy, $C_3$-$C_8$cyoloalkenyl or by a cyano, -$COOR_{10}$, $C_1$-$C_4$alkylcarbamoyl or piperidinocarbamoyl radical; $R_3$ and $R_4$ further represent $C_3$-$C_8$alkynyl which is unsubstituted or substituted by U, or $R_3$ and $R_4$ represent a radical -$(E)_m$-U or -$(E)_m$-Q; or $R_3$ and $R_4$ together with the nitrogen atom to which they are bonded also form a saturated or unsaturated heterocycle with 5 to 9 ring members which may be interrupted once or several times by oxygen, sulfur, nitrogen, -NH-, -$NC_1$-$C_4$alkyl, -CO- or -$C(OR_7)OR_8$- and which may be substituted by halogen, cyano, $C_1$-$C_8$alkoxy, amino, $C_1$-$C_4$alkylamino, di-$C_1$-$C_4$alkylamino or by a radical -$COOR_{10}$; $R_5$ and $R_6$ independently of each other represent hydroxy, $C_1$-$C_4$alkyl or $C_2$-$C_4$alkoxy; $R_7$ and $R_8$ independently of each other represent $C_1$-$C_4$alkyl or $R_7$ and $R_8$ together form a 2- to 4-membered alkylene chain, $R_9$ and $R_{10}$ independently of each other represent hydrogen, $C_1$-$C_3$alkyl, $C_3$-$C_8$cycloalkyl, $C_3$-$C_8$alkenyl, $C_3$-$C_8$cycloalkenyl, $C_3$-$C_8$alkynyl, $C_2$-$C_8$alkoxyalkyl, $C_4$-$C_8$alkoxyalkoxyalkyl, $C_1$-$C_4$haloalkyl-$(C_1$-$C_3alkyl)_m$U, -$(C_1$-$C_3alkyl)_m$Q, $C_1$-$C_4$haloalkoxy or $C_1$-$C_4$haloalkoxy-$C_1$-$C_4$alkyl; U is a phenyl or naphthyl radical which is unsubstituted or mono- or poly-substituted by halogen, $C_1$-$C_4$alkyl, -Y-$C_1$-$C_4$alkyl, $C_1$-$C_4$haloalkyl, $C_1$-$C_4$haloalkoxy, cyano, nitro, -COOH, -$COOR_7$, -$CONH_2$, -$CONHR_7$, -$CON(R_7)_2$, $SO_2NHR_7$, $SO_2N(R_7)_2$, pyrrolidino, piperidino, pyrrolidinocarbonyl or by piperidinocarbonyl; E is a $C_1$-$C_8$alkylene or $C_2$-$C_8$alkenylene chain which is unsubstituted or substituted by halogen, $C_1$-$C_4$alkoxy, $C_1$-$C_4$alkylthio, $C_1$-$C_4$haloalkoxy or by a radical -$CO(O)_mR_{10}$, -$(CO)_m$-A or $(CO)_m$Q and/or interrupted by a -CO- or -$C(OR_7)OR_8$- member; Q is a saturated or unsaturated heterocycle with 5 to 12 ring members which may contain 1 to 4 hetero atoms or a sulfinyl or sulfonyl group in combination with 1 or 2 hetero atoms and which may be interrupted by one or two carbonyl groups and may be benzofused, for the control or prevention of infestation of cultivated plants by pathogenic microorganisms.

**2.** The use according to claim 1 of a 2-chloro-4-trifluoromethylthiazole-5-carboxylic acid derivative of formula Ib

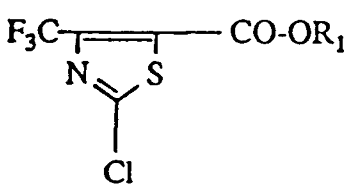

(Ib),

wherein $R_1$ has the meaning given in claim 1.

**3.** A 2-chloro-4-trifluoromethylthiazole-5-carboxylic acid derivative of formula Ib according to claim 2, wherein $R_1$ represents a tetrahydropyran-2-ylmethyl, 2,2-dimethyl-1,3-dioxolan-4-ylmethyl, 1,2-dihydrobenz-1,4-dioxan-2-ylmethyl, thiophen-2-ylmethyl, 3,4-methylenedioxybenzyl, 5-methylthiazol-4-ylethyl, para-tolyl-eth-1-yl, bornyl, norbornyl, fenchyl, menthyl, cyanoethyl, phenoxyethyl, methylsulfonylethyl, phenylthioethyl, ethoxycarbonylmethyl, ethoxycarbonyleth-1-yl, 5,5-dimethyltetrahydrofuran-2-on-3-yl, 2-oxopyrrolinomethyl, $\alpha$-methoxycarbonylbenzyl, $\alpha$-cyanobenzyl, $\alpha$-benzoylbenzyl, $\alpha$-methoxycarbonyl-$\alpha$-phenylbenzyl or morpholinomethyl radical.

**4.** A 2-chloro-4-trifluoromethylthiazole-5-carboxylic acid derivative of formula Ib according to claim 2, wherein $R_1$ represents a $C_1$-$C_{12}$alkyl radical substituted by -$(X)_m$-CX-A, wherein A, m and X have the meanings given in claim 1.

**5.** A 2-chloro-4-trifluoromethylthiazole-5-carboxylic acid derivative of formula Ib according to claim 4, wherein m is zero, X is oxygen and A has the meaning given in claim 1.

**6.** A 2-chloro-4-trifluoromethylthiazole-5-carboxylic acid derivative of formula Ib according to claim 2, wherein $R_1$ is an azepinomethyl, N-(2,6-dimethylphenyl)-N-(methoxycarbonyleth-1-yl)-carbamoylmethyl or 2-piperidinoeth-1-yl radical.

**7.** The use according to claim 1 of a 2-chloro-4-trifluoromethylthiazole-5-carboxylic acid derivative of formula Ic

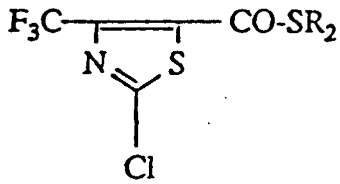

(Ic),

wherein $R_2$ has the meaning given in claim 1, for the control or prevention of infestation of cultivated plants by pathogenic microorganisms.

**8.** A 2-chloro-4-trifluoromethylthiazole-5-carboxylic acid derivative of formula Id

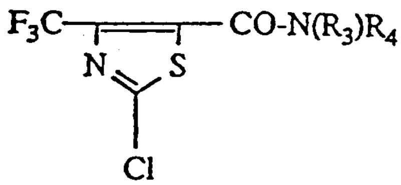

wherein one of $R_3$ and $R_4$ represents hydrogen or $C_1$-$C_8$alkyl which is unsubstituted and the other or both represent(s) $C_3$-$C_8$cycloalkyl which is unsubstituted, or $C_1$-$C_8$alkyl or $C_3$-$C_8$cycloalkyl which is substituted by $C_1$-$C_8$alkoxy, $C_2$-$C_8$alkoxyalkoxy, $C_1$-$C_8$alkylthio, cyano, a radical -$COOR_{10}$, $C_1$-$C_4$alkylcarbamoyl, di-$C_1$-$C_4$alkylcarbamoyl, $C_1$-$C_4$alkylamino, di-$C_1$-$C_4$alkylamino, piperidinocarbonyl, pyrrolidinocarbonyl, piperidino or pyrrolidino; $R_3$ and $R_4$ further represent $C_3$-$C_8$alkenyl or $C_3$-$C_8$cycloalkenyl which is unsubstituted or substituted by halogen, $C_1$-$C_8$alkoxy, $C_3$-$C_8$cycloalkenyl or a cyano, -$COOR_{10}$, $C_1$-$C_4$alkylcarbamoyl, di-$C_1$-$C_4$alkylcarbamoyl, U, pyrrolidinocarbamoyl or piperidinocarbamoyl radical; $R_3$ and $R_4$ further represent $C_3$-$C_8$alkynyl which is unsubstituted or substituted by U, or $R_3$ and $R_4$ represent a radical -$(E)_m$-U or -$(E)_m$-Q; or $R_3$ and $R_4$ together with the nitrogen atom to which they are bonded also form a saturated or unsaturated heterocycle with 5 to 9 ring members which may be interrupted once or several times by oxygen, sulfur, nitrogen, -NH-, -N$C_1$-$C_4$alkyl, -CO- or -C($OR_7$)$OR_8$- and which may be substituted by halogen, cyano, $C_1$-$C_8$alkoxy, amino, $C_1$-$C_4$alkylamino, di-$C_1$-$C_4$alkylamino or by a radical -$COOR_{10}$.

**9.** A fungicidal composition that comprises as active ingredient a 2-chloro-4-trifluoromethylthiazole-5-carboxylic acid derivative of formula Id

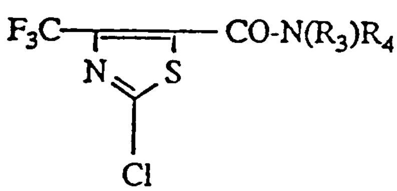

(Id),

wherein $R_3$ and $R_4$ have the meanings given in claim 8.

**10.** The use according to claim 1 of a 2-chloro-4-trifluoromethylthiazole-5-carboxylic acid derivative of formula Id

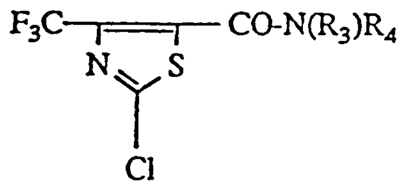

(Id),

wherein $R_3$ and $R_4$ have the meanings given in claim 8, for the control or prevention of infestation of cultivated plants by pathogenic microorganisms.

**11.** A 2-chloro-4-trifluoromethylthiazole-5-carboxylic acid hydrazide according to claim 1 of formula Ie

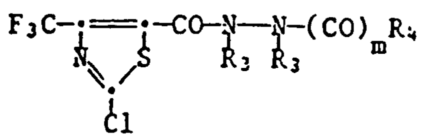

(Ie),

wherein m and $R_3$ and $R_4$ independently of each other have the meanings given in claim 8.

**12.** A 2-chloro-4-trifluoromethylthiazole-5-carboxylic acid hydrazine according to claim 1 of formula If

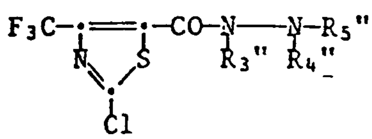

(If),

wherein $R_3$", $R_4$" and $R_5$" independently of one another represent hydrogen, $C_1$-$C_8$alkyl, $C_3$-$C_8$cycloalkyl, phenyl or benzyl, phenyl and benzyl being unsubstituted or substituted by halogen, $C_1$-$C_4$alkyl, $C_1$-$C_4$haloalkyl, $C_1$-$C_4$alkoxy, $C_1$-$C_4$haloalkyl, nitro, carboxy, $C_1$-$C_4$alkoxycarbonyl, carbamoyl or by methylcarbamoyl, $R_3$" and $R_4$" together also represent a 4- or 5-membered alkylene chain which may be interrupted by oxygen, sulfur, imino or $C_1$-$C_4$alkylimino and which may be mono- or polysubstituted by $C_1$-$C_4$alkyl.

**13.** A 2-chloro-4-trifluoromethylthiazolecarboxylic acid amide of formula Id according to claim 1, wherein -N($R_3$)$R_4$ represents a -N-2,4-dichlorobenzyl-N-methylcarbamoyl, -N-benzyl-N-isopropylcarbamoyl, -N-cyclohexyl-N-methoxycarbamoylethylcarbamoyl, -N-2,6-dimethylphenyl-N-methoxycarbamoyleth-1-yl-carbamoyl or -N-(1-cyanopent-1-yl)-N-methylcarbamoyl radical.

**14.** A method of controlling or preventing infestation of cultivated plants by phytopathogenic microorganisms which comprises applying a compound of formula Ia defined according to claim 1 to the plant or the locus thereof.

**15.** The use of a compound of formula I according to claim 1 or of a composition comprising it for the control and/or prevention of infestation by microorganisms.

**16.** The use according to claim 15, wherein the microorganisms are phytopathogenic fungi.

**17.** A method according to claim 14, wherein the parts of the plants are the seeds.

**18.** A method according to claim 14, which comprises treating rice plants.

**19.** The use according to claim 15, wherein the microorganisms are soil-borne phytopathogenic bacteria.

**Claims for the following Contracting State : ES**

1. The use of a 2-chloro-4-trifluoromethylthiazole-5-carboxylic acid derivative of formula Ia

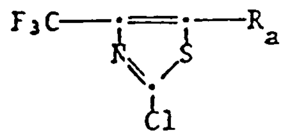

(Ia),

wherein $R_a$ is a cyano, $-COXR_1$, $-CONR_3R_4$ or -CO-D radical, X is oxygen or sulfur, $R_1$ is hydrogen, or $C_1$-$C_{18}$alkyl or $C_3$-$C_{18}$cycloalkyl which is unsubstituted or substituted by halogen, a group $-YR_2$, A, nitro, $-N(R_3)COA$, $-[N(R_3)]_m-C(A)=NC_1-C_4alkyl$, $-[N(R_3)]_m-C(A)=NH$, $-[N(R_3)]_m-CO-N\{(CO)_mR_3\}-N(R_3)$ or $-[N(R_3)]_m-CON[(CO)_m-R_3]-N(R_3)_m-(CO_m)R_4$, in which groups one of the indices m must be zero, furthermore the $C_1$-$C_{18}$alkyl or $C_3$-$C_{18}$cycloalkyl radical can be substituted by cyano or by a radical $-C-(X)_m-XR_{10}$, $-XCXR_{10}$, $-(X)_m-CXA$, $-(X)CXN(R_3)N(R_3)R_4$, $-CHA-COOR_{10}$, $-C(OR_7)(OR_8)R_9$, $PO(R_5)R_6$, $C_3$-$C_8$cycloalkyl or $C_5$-$C_8$cycloalkenyl; $R_1$ further represents $C_3$-$C_8$alkenyl or $C_3$-$C_{18}$cycloalkenyl which is unsubstituted or substituted by halogen, $C_1$-$C_4$alkoxy, $C_1$-$C_4$alkylthio, $C_1$-$C_4$haloalkoxy, $C_1$-$C_4$haloalkyl or by a radical $CO(O)_m-R_{10}$, -COA, $-CON(A)R_3$ or $PO(R_5)R_6$-; $R_1$ further represents $C_3$-$C_8$alkynyl which is unsubstituted or a radical $-(E)_mU$ or $-(E)_mQ$, $R_2$ represents $C_1$-$C_8$alkyl or $C_3$-$C_8$cycloalkyl which is unsubstituted or substituted by $C_1$-$C_8$alkoxy, $C_1$-$C_8$alkylthio, $C_3$-$C_8$alkoxyalkoxy, halogen, cyano or by a radical $-CX(X)_mR_{10}$, $-(X)_m-CX-A$, $-(X)_mCXR_{10}$, $N(R_3)COA$, $-[N(R_3)]_m-C(A)=NH$, $-[N(R_3)]_m-C(A)=NC_1$-$C_4$alkyl A, -X-U or -XQ; $R_2$ further represents $C_3$-$C_8$alkenyl or $C_3$-$C_8$cycloalkenyl which is unsubstituted or substituted by halogen, or represents a radical $-(E)_mU$ or $-(E)_mQ$; m is zero or one; Y is oxygen, sulfur, SO or $SO_2$; A is a radical $-N(R_3)R_4$; D is a radical $-N(R_3)N(R_4)(CO)_mR_3$; $R_3$ and $R_4$ independently of each other represent hydrogen, or $C_1$-$C_8$alkyl or $C_3$-$C_8$cycloalkyl which is unsubstituted or substituted by $C_1$-$C_8$alkoxy, $C_2$-$C_8$alkoxyalkoxy, $C_1$-$C_8$alkylthio, cyano, a radical $-COOR_{10}$, $C_1$-$C_4$alkylcarbamoyl, di-$C_1$-$C_4$alkylcarbamoyl, $C_1$-$C_4$alkylamino, di-$C_1$-$C_4$alkylamino, piperidinocarbonyl, pyrrolidinocarbonyl, piperidino or pyrrolidino; $R_3$ and $R_4$ further represent $C_3$-$C_8$alkenyl or $C_3$-$C_8$cycloalkenyl which is unsubstituted or substituted by halogen, $C_1$-$C_8$alkoxy, $C_3$-$C_8$cycloalkenyl or by a cyano, $-COOR_{10}$, $C_1$-$C_4$alkylcarbamoyl or piperidinocarbamoyl radical; $R_3$ and $R_4$ further represent $C_3$-$C_8$alkynyl which is unsubstituted or substituted by U, or $R_3$ and $R_4$ represent a radical $-(E)_m-U$ or $-(E)_m-Q$; or $R_3$ and $R_4$ together with the nitrogen atom to which they are bonded also form a saturated or unsaturated heterocycle with 5 to 9 ring members which may be interrupted once or several times by oxygen, sulfur, nitrogen, -NH-, $-NC_1$-$C_4$alkyl, -CO- or $-C(OR_7)OR_8$- and which may be substituted by halogen, cyano, $C_1$-$C_8$alkoxy, amino, $C_1$-$C_4$alkylamino, di-$C_1$-$C_4$alkylamino or by a radical $-COOR_{10}$; $R_5$ and $R_6$ independently of each other represent hydroxy, $C_1$-$C_4$alkyl or $C_2$-$C_4$alkoxy; $R_7$ and $R_8$ independently of each other represent $C_1$-$C_4$alkyl or $R_7$ and $R_8$ together form a 2- to 4-membered alkylene chain, $R_9$ and $R_{10}$ independently of each other represent hydrogen, $C_1$-$C_3$alkyl, $C_3$-$C_8$cycloalkyl, $C_3$-$C_8$alkenyl, $C_3$-$C_8$cycloalkenyl, $C_3$-$C_8$alkynyl, $C_2$-$C_8$alkoxyalkyl, $C_4$-$C_8$alkoxyalkoxyalkyl, $C_1$-$C_4$haloalkyl-$(C_1$-$C_3alkyl)_mU$, $-(C_1$-$C_3alkyl)_mQ$, $C_1$-$C_4$haloalkoxy or $C_1$-$C_4$haloalkoxy$C_1$-$C_4$alkyl; U is a phenyl or naphthyl radical which is unsubstituted or mono- or poly-substituted by halogen, $C_1$-$C_4$alkyl, $-Y-C_1$-$C_4$alkyl, $C_1$-$C_4$haloalkyl, $C_1$-$C_4$haloalkoxy, cyano, nitro, -COOH, $-COOR_7$, $-CONH_2$, $-CONHR_7$, $-CON(R_7)_2$, $SO_2NHR_7$, $SO_2N(R_7)_2$, pyrrolidino, piperidino, pyrrolidinocarbonyl or by piperidinocarbonyl; E is a $C_1$-$C_8$alkylene or $C_2$-$C_8$alkenylene chain which is unsubstituted or substituted by halogen, $C_1$-$C_4$alkoxy, $C_1$-$C_4$alkylthio, $C_1$-$C_4$haloalkoxy or by a radical $-CO(O)_mR_{10}$, $-(CO)_m-A$ or $-(CO)_mQ$ and/or interrupted by a -CO- or $-C(OR_7)OR_8$- member; Q is a saturated or unsaturated heterocycle with 5 to 12 ring members which may contain 1 to 4 hetero atoms or a sulfinyl or sulfonyl group in combination with 1 or 2 hetero atoms and which may be interrupted by one or two carbonyl groups and may be benzofused, for the control or prevention of infestation of cultivated plants by pathogenic microorganisms.

2. The use according to claim 1 of a 2-chloro-4-trifluoromethylthiazole-5-carboxylic acid derivative of formula Ib

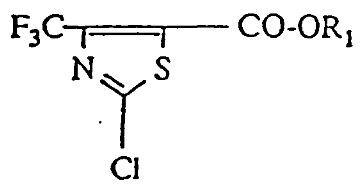

(Ib),

wherein $R_1$ has the meaning given in claim 1.

**3.** The use according to claim 1 of a 2-chloro-4-trifluoromethylthiazole-5-carboxylic acid derivative of formula Ib according to claim 2, wherein $R_1$ represents a tetrahydropyran-2-ylmethyl, 2,2-dimethyl-1,3-dioxolan-4-ylmethyl, 1,2-dihydrobenz-1,4-dioxan-2-ylmethyl, thiophen-2-ylmethyl, 3,4-methylenedioxybenzyl, 5-methylthiazol-4-ylethyl, para-tolyl-eth-1-yl, bornyl, norbornyl, fenchyl, menthyl, cyanoethyl, phenoxyethyl, methylsulfonylethyl, phenylthioethyl, ethoxycarbonylmethyl, ethoxycarbonyleth-1-yl, 5,5-dimethyltetrahydrofuran-2-on-3-yl, 2-oxopyrrolinomethyl, α-methoxycarbonylbenzyl, α-cyanobenzyl, α-benzoylbenzyl, α-methoxycarbonyl-α-phenylbenzyl or morpholinomethyl radical.

**4.** The use according to claim 1 of a 2-chloro-4-trifluoromethylthiazole-5-carboxylic acid derivative of formula Ib according to claim 2, wherein $R_1$ represents a $C_1$-$C_{12}$alkyl radical substituted by $-(X)_m$-CX-A, wherein A, m and X have the meanings given in claim 1.

**5.** The use according to claim 1 of a 2-chloro-4-trifluoromethylthiazole-5-carboxylic acid derivative of formula Ib according to claim 4, wherein m is zero, X is oxygen and A has the meaning given in claim 1.

**6.** The use according to claim 1 of a 2-chloro-4-trifluoromethylthiazole-5-carboxylic acid derivative of formula Ib according to claim 2, wherein $R_1$ is an azepinomethyl, N-(2,6-dimethylphenyl)-N-(methoxycarbonyleth-1-yl)-carbamoylmethyl or 2-piperidinoeth-1-yl radical.

**7.** The use according to claim 1 of a 2-chloro-4-trifluoromethylthiazole-5-carboxylic acid derivative of formula Ic

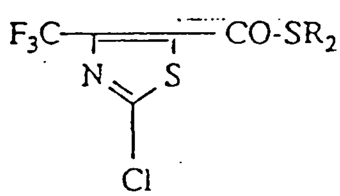

(Ic),

wherein $R_2$ has the meaning given in claim 1, for the control or prevention of infestation of cultivated plants by pathogenic microorganisms.

**8.** A fungicidal composition that comprises as active ingredient a 2-chloro-4-trifluoromethylthiazole-5-carboxylic acid derivative of formula Id

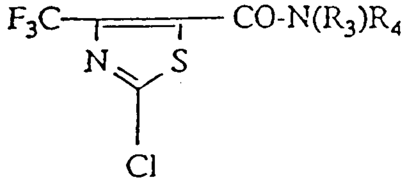

(Id),

wherein one of $R_3$ and $R_4$ represents hydrogen or $C_1$-$C_8$alkyl which is unsubstituted and the other or both represent(s) $C_3$-$C_8$cycloalkyl which is unsubstituted, or $C_1$-$C_8$alkyl or $C_3$-$C_8$cycloalkyl which is substituted by $C_1$-$C_8$alkoxy, $C_2$-$C_8$alkoxyalkoxy, $C_1$-$C_8$alkylthio, cyano, a radical $-COOR_{10}$, $C_1$-$C_4$alkylc-

arbamoyl, di-$C_1$-$C_4$alkylcarbamoyl, $C_1$-$C_4$alkylamino, di-$C_1$-$C_4$alkylamino, piperidinocarbonyl, pyrrolidinocarbonyl, piperidino or pyrrolidino; $R_3$ and $R_4$ further represent $C_3$-$C_8$alkenyl or $C_3$-$C_8$cycloalkenyl which is unsubstituted or substituted by halogen, $C_1$-$C_8$alkoxy, $C_3$-$C_8$cycloalkenyl or a radical cyano, $-COOR_{10}$, $C_1$-$C_4$alkylcarbamoyl, di-$C_1$-$C_4$alkylcarbamoyl, U, pyrrolidinocarbamoyl or piperidinocarbamoyl; $R_3$ and $R_4$ further represent $C_3$-$C_8$alkynyl which is unsubstituted or substituted by U, or $R_3$ and $R_4$ represent a radical $-(E)_m-U$ or $-(E)_m-Q$; or $R_3$ and $R_4$ together with the nitrogen atom to which they are bonded also form a saturated or unsaturated heterocycle with 5 to 9 ring members which may be interrupted once or several times by oxygen, sulfur, nitrogen, -NH-, $-NC_1$-$C_4$alkyl, -CO- or $-C(OR_7)OR_8-$ and which may be substituted by halogen, cyano, $C_1$-$C_8$alkoxy, amino, $C_1$-$C_4$alkylamino, di-$C_1$-$C_4$alkylamino or by a radical $-COOR_{10}$.

**9.** The use according to claim 1 of a 2-chloro-4-trifluoromethylthiazole-5-carboxylic acid derivative of formula Id

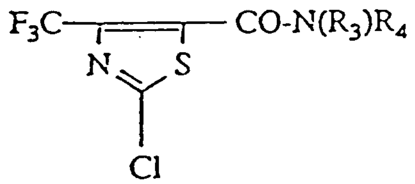

(Id),

wherein $R_3$ and $R_4$ have the meanings given in claim 8, for the control or prevention of infestation of cultivated plants by pathogenic microorganisms.

**10.** A method of controlling or preventing infestation of cultivated plants by phytopathogenic microorganisms which comprises applying a compound of formula Ia defined according to claim 1 to the plant or the locus thereof.

**11.** The use of a compound of formula I according to claim 1 or of a composition comprising it for the control and/or prevention of infestation by microorganisms.

**12.** The use according to claim 11, wherein the microorganisms are phytopathogenic fungi.

**13.** A method according to claim 10, wherein the parts of the plants are the seeds.

**14.** A method according to claim 10, which comprises treating rice plants.

**15.** The use according to claim 11, wherein the microorganisms are soil-borne phytopathogenic bacteria.

**Revendications**

**Revendications pour les Etats contractants suivants : AT, BE, CH, DE, FR, GB, IT, LI, NL**

**1.** Utilisation de dérivés d'acide 2-chloro-4-trifluorométhyl-thiazole-5-carboxylique de formule Ia

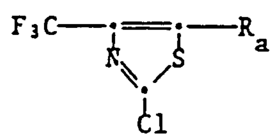

(Ia)

dans laquelle

$R_a$ représente un radical cyano, $-COXR_1$, $-CONR_3R_4$ ou -CO-D,

X représente un atome d'oxygène ou de soufre,

$R_1$ représente un atome d'hydrogène ou un groupe alkyle en $C_1$-$C_{18}$ ou cycloalkyle en $C_3$-$C_{18}$ qui n'est pas substitué ou est substitué par un halogène, un groupe $-YR_2$, A, nitro, $-N(R_3)COA$, $-[N(R_3)]_m-C(A)=N$-alkyle($C_1$-$C_4$), $-[N(R_3)]_m-C(A)=NH$ ou $-[N(R_3)]_m-$

CO-N[(CO)$_m$R$_3$]-N(R$_3$), -[N(R$_3$)]$_m$-CON[(CO)$_m$-R$_3$]-N(R$_3$)$_m$-(CO)$_m$R$_4$, dans lequel radical l'un des indices m doit être zéro, en outre, le radical alkyle en $C_1$-$C_{18}$ ou cycloalkyle en $C_3$-$C_{18}$ peut être substitué par un groupe cyano, un radical -C(X)$_m$-XR$_{10}$, -XCXR$_{10}$, -(X)$_m$-CXA, -(X)CXN(R$_3$)N(R$_3$)R$_4$, -CHA-COOR$_{10}$, -C(OR$_7$)(OR$_8$)R$_9$, -PO(R$_5$)R$_6$, cycloalkyle en $C_3$-$C_8$ ou cycloalcényle en $C_5$-$C_8$; R$_1$ est en outre un groupe alcényle en $C_3$-$C_8$ ou cycloalcényle en $C_3$-$C_{18}$, qui n'est pas substitué ou est substitué par des substituants halogènes, alcoxy en $C_1$-$C_4$, alkyl($C_1$-$C_4$)-thio, halogénoalcoxy en $C_1$-$C_4$, halogénoalkyle en $C_1$-$C_4$ ou un radical -CO(O)$_m$-R$_{10}$, -COA, -CON(A)R$_3$ ou -PO(R$_5$)R$_6$, R$_1$ est en outre un radical alcynyle en $C_3$-$C_8$ qui n'est pas substitué ou un radical -(E)-$_m$U ou -(E)$_m$-Q, R$_2$ est un radical alkyle en $C_1$-$C_8$ ou cycloalkyle en $C_3$-$C_8$ qui n'est pas substitué ou est substitué par des substituants alcoxy en $C_1$-$C_8$, alkyl($C_1$-$C_8$)-thio, alcoxy($C_3$-$C_8$)-alcoxy, halogène, cyano, ou un radical -CX(X)$_m$R$_{10}$, -(X)$_m$-CX-A, -(X)-$_m$CXR$_{10}$, N(R$_3$)COA, -[N(R$_3$)]$_m$-C(A) = NH, -[N(R$_3$)]$_m$-C(A) = N-alkyle($C_1$-$C_4$), A, -X-U ou -XQ, R$_2$ représente en outre un radical alcényle en $C_3$-$C_8$ ou cycloalcényle en $C_3$-$C_8$ qui n'est pas substitué ou est substitué par un halogène, ou un radical -(E)$_m$U ou -(E)-$_m$Q;

| | |
|---|---|
| m | est 0 ou 1; |
| Y | est un atome d'oxygène, de soufre, SO ou $SO_2$; |
| A | est un radical -N(R$_3$)R$_4$; |
| D | est un radical -N(R$_3$)N(R$_4$)(CO)$_m$R$_3$; |
| R$_3$ et R$_4$ | sont chacun, indépendamment l'un de l'autre, un atome d'hydrogène ou un radical alkyle en $C_1$-$C_8$ ou cycloalkyle en $C_3$-$C_8$ qui n'est pas substitué ou est substitué par un groupe alcoxy en $C_1$-$C_8$, alcoxy($C_2$-$C_8$)-alcoxy, alkyl($C_1$-$C_8$)-thio, cyano, -COOR$_{10}$, alkyl($C_1$-$C_4$)-carbamoyle, dialkyl($C_1$-$C_4$)-carbamoyle, alkyl($C_1$-$C_4$)-amino, dialkyl($C_1$-$C_4$)-amino, pipéridinocarbonyle, pyrrolidinocarbonyle, pipéridino ou pyrrolidino; R$_3$ et R$_4$ sont en outre un radical alcényle en $C_3$-$C_8$ ou cycloalcényle en $C_3$-$C_8$, qui n'est pas substitué ou est substitué par un atome d'halogène ou par un groupe alcoxy en $C_1$-$C_8$, cycloalcényle en $C_3$-$C_8$, cyano, -COOR$_{10}$, alkyl($C_1$-$C_4$)-carbamoyle, dialkyl($C_1$-$C_4$)- carbamoyle, U, pyrrolidinocarbamoyle ou pipéridinocarbamoyle, R$_3$ et R$_4$ sont en outre un radical alcynyle en $C_3$-$C_8$ qui n'est pas substitué ou est substitué par U, ou R$_3$ et R$_4$ représentent un radical -(E)$_m$-U ou -(E)$_m$-Q. |
| R$_3$ et R$_4$ | forment ensemble, avec l'atome d'azote auquel ils sont liés, également un hétérocycle saturé ou insaturé à 5-9 chaînons, qui peut être une ou plusieurs fois interrompu par un ou des atomes d'oxygène, de soufre, d'azote ou groupes -NH-, -N-alkyle($C_1$-$C_4$), -CO- ou -C(OR$_7$)OR$_8$- et substitué par un ou des atomes d'halogène ou groupes cyano, alcoxy en $C_1$-$C_8$, amino, alkyl($C_1$-$C_4$)-amino, dialkyl($C_1$-$C_4$)-amino ou -COOR$_{10}$; |
| R$_5$ et R$_6$ | sont chacun, indépendamment l'un de l'autre, un groupe hydroxy, alkyle en $C_1$-$C_4$ ou alcoxy en $C_2$-$C_4$; |
| R$_7$ et R$_8$ | sont chacun, indépendamment l'un de l'autre, un groupe alkyle en $C_1$-$C_4$, ou |
| R$_7$ et R$_8$ | forment ensemble une chaîne alkylène à 2-4 chaînons, |
| R$_9$ et R$_{10}$ | sont chacun, indépendamment l'un de l'autre, un atome d'hydrogène ou un groupe alkyle en $C_1$-$C_3$, cycloalkyle en $C_3$-$C_8$, alcényle en $C_3$-$C_8$, cycloalcényle en $C_3$-$C_8$, alcynyle en $C_3$-$C_8$, alcoxy($C_2$-$C_8$)-alkyle, alcoxy($C_4$-$C_8$)-alcoxyalkyle, halogénoalkyle en $C_1$-$C_4$, -[alkyl($C_1$-$C_3$)]$_m$U, -[alkyl($C_1$-$C_3$)]$_m$Q, halogénoalcoxy en $C_1$-$C_4$, halogénoalcoxy-($C_1$-$C_4$)-alkyle($C_1$-$C_4$); |
| U | est un radical phényle ou naphtyle qui n'est pas substitué ou est substitué une ou plusieurs fois par un ou des substituants halogène, alkyle en $C_1$-$C_4$, -Y-alkyle($C_1$-$C_4$), halogénoalkyle en $C_1$-$C_4$, halogénoalcoxy en $C_1$-$C_4$, cyano, nitro, -COOH, -COOR$_7$, -$CONH_2$, -CONHR$_7$, -CON(R$_7$)$_2$, $SO_2$NHR$_7$, $SO_2$N(R$_7$)$_2$, pyrrolidino, pipéridino, pyrrolidinocarbonyle ou pipéridinocarbonyle; |
| E | est une chaîne alkylène en $C_1$-$C_8$, alcénylène en $C_2$-$C_8$, qui n'est pas substituée ou est substituée par un atome d'halogène ou par un groupe alcoxy en $C_1$-$C_4$, alkyl($C_1$-$C_4$)-thio, halogénoalcoxy en $C_1$-$C_4$, -CO(O)$_m$R$_{10}$, (CO)$_m$-A ou -(CO)$_m$Q, et/ou est interrompue par un chaînon -CO- ou -C(OR$_7$)OR$_8$-; |
| Q | est un hétérocycle saturé ou insaturé à 5-12 chaînons, qui peut contenir 1-4 hétéroatomes ou un groupe sulfinyle ou sulfonyle, également conjointement avec 1-2 hétéroatomes, qui peut être interrompu par 1 ou 2 groupes carbonyle et soudé à un noyau benzénique, |

pour le traitement ou la prévention d'une attaque de plantes cultivées par des micro-organismes pathogènes.

2. Utilisation selon la revendication 1 de dérivés d'acide 2-chloro-4-trifluorométhyl-thiazole-5-carboxylique de formule Ib

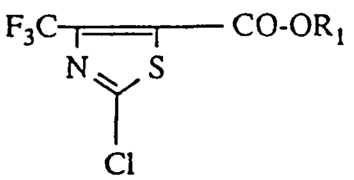

(Ib)

dans laquelle $R_1$ a la signification donnée dans la revendication 1.

3. Dérivés d'acide 2-chloro-4-trifluorométhyl-thiazole-5-carboxylique de formule Ib selon la revendication 2, dans lesquels $R_1$ représente le radical
tétrahydropyranne-2-ylméthyle,
2,2-diméthyl-1,3-dioxolanne-4-ylméthyle,
1,2-dihydrobenz-1,4-dioxanne-2-ylméthyle,
thiophène-2-ylméthyle,
3,4-méthylènedioxybenzyle,
5-méthylthiazole-4-yléthyle,
p-tolyl-éth-1-yle,
bornyle,
norbornyle,
fenchyle,
menthyle,
cyanoéthyle,
phénoxyéthyle,
méthylsulfonyléthyle,
phénylthioéthyle,
éthoxycarbonylméthyle,
éthoxycarbonyléth-1-yle,
5,5-diméthyl-tétrahydrofuranne-2-one-3-yle,
2-oxopyrrolinométhyle,
$\alpha$-méthoxycarbonylbenzyle,
$\alpha$-cyanobenzyle,
$\alpha$-benzoylbenzyle ou
$\alpha$-méthoxycarbonyl-$\alpha$-phénylbenzyle ou
le radical morpholinométhyle.

4. Dérivés d'acide 2-chloro-4-trifluorométhyl-thiazole-5-carboxylique de formule Ib selon la revendication 2, dans lesquels $R_1$ représente un radical alkyle en $C_1$-$C_{12}$ qui est substitué par $-(X)_m-CX-A$, tandis que A, m et X ont les significations données dans la revendication 1.

5. Dérivés d'acide 2-chloro-4-trifluorométhyl-thiazole-5-carboxylique de formule Ib selon la revendication 4, dans lesquels m est zéro et X représente un atome d'hydrogène, tandis que A a la signification donnée dans la revendication 1.

6. Dérivés d'acide 2-chloro-4-trifluorométhyl-thiazole-5-carboxylique de formule Ib selon la revendication 2, dans lesquels $R_1$ représente le radical azépinométhyle, N-(2,6-diméthylphényl)-N-(méthoxycarbonyléth-1-yl)-carbamoylméthyle ou 2-pipéridino-éth-1-yle.

7. Utilisation selon la revendication 1 des dérivés d'acide 2-chloro-4-trifluorométhylthiazole-5-carboxylique de formule Ic

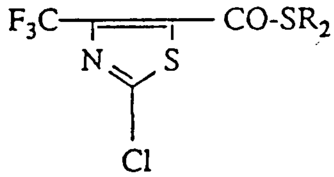

(Ic)

dans laquelle $R_2$ a la signification donnée dans la revendication 1, pour la lutte contre ou la prévention d'une attaque de plantes cultivées par des micro-organismes pathogènes.

**8.** Dérivés d'acide 2-chloro-4-trifluorométhyl-thiazole-5-carboxylique de formule Id

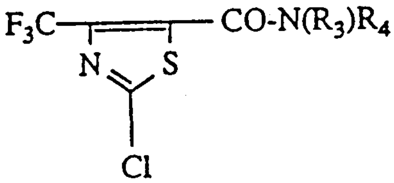

dans laquelle l'un des radicaux $R_3$ et $R_4$ représente un atome d'hydrogène ou un groupe alkyle en $C_1$-$C_8$ non substitué, l'autre ou les deux représente(nt) un radical cycloalkyle en $C_3$-$C_8$ qui n'est pas substitué ou un radical alkyle en $C_1$-$C_8$ ou cycloalkyle en $C_3$-$C_8$ qui est substitué par un groupe alcoxy en $C_1$-$C_8$, alcoxy($C_2$-$C_8$)-alcoxy, alkyl($C_1$-$C_8$)-thio, cyano, $-COOR_{10}$, alkyl($C_1$-$C_4$)-carbamoyle, dialkyl-($C_1$-$C_4$)-carbamoyle, alkyl($C_1$-$C_4$)-amino, dialkyl($C_1$-$C_4$)-amino, pipéridinocarbonyle, pyrrolidinocarbonyle, pipéridino ou pyrrolidino; $R_3$ et $R_4$ sont en outre un radical alcényle en $C_3$-$C_8$ ou cycloalcényle en $C_3$-$C_8$ qui n'est pas substitué ou est substitué par un atome d'halogène ou un groupe alcoxy en $C_1$-$C_8$, cycloalcényle en $C_3$-$C_8$, cyano, $-COOR_{10}$, alkyl($C_1$-$C_4$)-carbamoyle, dialkyl($C_1$-$C_4$)-carbamoyle, U, pyrrolidinocarbamoyle, ou pipéridinocarbamoyle, $R_3$ et $R_4$ sont en outre un radical alcynyle en $C_3$-$C_8$ qui n'est pas substitué ou est substitué par U, ou $R_3$ et $R_4$ représentent un radical $-(E)_m$-U ou $-(E)_m$-Q, et $R_3$ et $R_4$ forment également, conjointement avec l'atome d'azote auquel ils sont liés, un hétérocycle saturé ou insaturé à 5-9 chaînons, qui peut être une ou plusieurs fois interrompu par des atomes d'oxygène, de soufre, d'azote ou des groupes -NH-, -N-alkyl($C_1$-$C_4$)-, -CO- ou $-C(OR_7)OR_8$- et substitué par un ou des substituants halogène, cyano, alcoxy en $C_1$-$C_8$, amino, alkyl($C_1$-$C_4$)-amino, dialkyl($C_1$-$C_4$)-amino ou $-COOR_{10}$.

**9.** Produit fongicide, contenant en tant que substance active un dérivé d'acide 2-chloro-4-trifluorométhyl-thiazole-5-carboxylique de formule Id

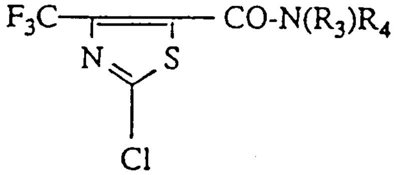

(Id)

dans laquelle $R_3$ et $R_4$ ont les significations données dans la revendication 8.

**10.** Utilisation selon la revendication 1 des dérivés d'acide 2-chloro-4-trifluorométhyl-thiazole-5-carboxylique de formule Id

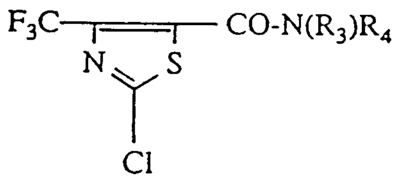

(Id)

dans laquelle $R_3$ et $R_4$ ont les significations données dans la revendication 8, pour la lutte contre ou la prévention d'une attaque de plantes cultivées par des micro-organismes pathogènes

**11.** Hydrazides d'acide 2-chloro-4-trifluorométhyl-thiazole-5-carboxylique selon la revendication 1, de formule Ie

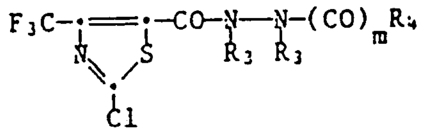

(Ie)

dans laquelle m, les radicaux $R_3$ et $R_4$ ont, indépendamment les uns des autres, l'une des significations données dans la revendication 8.

**12.** Hydrazides d'acide 2-chloro-4-trifluorométhyl-thiazole-5-carboxylique selon la revendication 1, de formule If

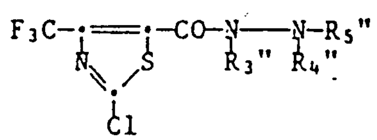

(If)

dans laquelle $R_3$", $R_4$" et $R_5$" représentent chacun, indépendamment les uns des autres, un atome d'hydrogène ou un radical alkyle en $C_1$-$C_8$, cycloalkyle en $C_3$-$C_8$, phényle ou benzyle, les radicaux phényle et benzyle n'étant pas substitués ou étant substitués par des substituants halogène, alkyle en $C_1$-$C_4$, halogénoalkyle en $C_1$-$C_4$, alcoxy en $C_1$-$C_4$, halogénoalcoxy en $C_1$-$C_4$, nitro, carboxy, alcoxy($C_1$-$C_4$)-carbonyle, carbamoyle, méthylcarbamoyle, ou $R_3$" et $R_4$" également forment ensemble une chaîne alkylène à 4-5 chaînons, qui peut être interrompue par un atome d'oxygène ou de soufre ou un groupe imino ou alkyl($C_1$-$C_4$)-imino et qui peut être une ou plusieurs fois substituée par un ou des groupes alkyle en $C_1$-$C_4$.

**13.** 2-chloro-4-trifluorométhyl-thiazole-carboxamides de formule 1d selon la revendication 1, dans lesquels -N($R_3$)$R_4$ représente le radical
-N-2,4-dichlorobenzyl-N-méthylcarbamoyle,
-N-benzyl-N-isopropylcarbamoyle,
-N-cyclohexyl-N-méthoxycarbamoyléthylcarbamoyle,
-N-2,6-diméthylphényl-N-méthoxycarbamoyléth-1-ylcarbamoyle ou
-N-(1-cyanopent-1-yl)-N-méthylcarbamoyle,

**14.** Procédé pour la lutte contre la prévention d'une attaque de plantes cultivées par des micro-organismes phytopathogènes, caractérisé en ce que l'on applique sur les plantes ou leur site un composé de formule Ia défini selon la revendication 1.

**15.** Utilisation de composés de formule I selon la revendication 1 ou de produits les contenant, pour la lutte contre et/ou la prévention d'une attaque par des micro-organismes.

**16.** Utilisation selon la revendication 15, caractérisée en ce que, en ce qui concerne les micro-organismes, il s'agit de champignons phytopathogènes.

**17.** Procédé selon la revendication 14, caractérisé en ce que les parties de plantes sont les graines.

**18.** Procédé selon la revendication 14, caractérisé en ce que l'on traite des plants de riz.

**19.** Utilisation selon la revendication 15, caractérisé en ce que, en ce qui concerne les micro-organismes, il s'agit de bactéries phytopathogènes du sol.

**Revendications pour l'Etat contractant suivant : ES**

**1.** Utilisation de dérivés d'acide 2-chloro-4-trifluorométhyl-thiazole-5-carboxylique de formule Ia

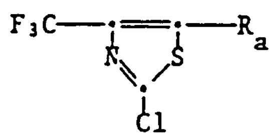

(Ia)

dans laquelle

$R_a$ représente un radical cyano, $-COXR_1$, $-CONR_3R_4$ ou -CO-D,

X représente un atome d'oxygène ou de soufre,

$R_1$ représente un atome d'hydrogène ou un groupe alkyle en $C_1$-$C_{18}$ ou cycloalkyle en $C_3$-$C_{18}$ qui n'est pas substitué ou est substitué par un halogène, un groupe $-YR_2$, A, nitro, $-N(R_3)COA$, $-[N(R_3)]_m-C(A)=N$-alkyle($C_1$-$C_4$), $-[N(R_3)]_m-C(A)=NH$ ou $-[N(R_3)]_m-CO-N[(CO)_mR_3]-N(R_3)$, $-[N(R_3)]_m-CON[(CO)_m-R_3]-N(R_3)_m-(CO)_mR_4$, dans lequel radical l'un des indices m doit être zéro, en outre, le radical alkyle en $C_1$-$C_{18}$ ou cycloalkyle en $C_3$-$C_{18}$ peut être substitué par un groupe cyano, un radical $-C(X)_m-XR_{10}$, $-XCXR_{10}$, $-(X)_m-CXA$, $-(X)CXN(R_3)N(R_3)R_4$, $-CHA-COOR_{10}$, $-C(OR_7)(OR_8)R_9$, $-PO(R_5)R_6$, cycloalkyle en $C_3$-$C_8$ ou cycloalcényle en $C_5$-$C_8$; $R_1$ est en outre un groupe alcényle en $C_3$-$C_8$ ou cycloalcényle en $C_3$-$C_{18}$, qui n'est pas substitué ou est substitué par des substituants halogènes, alcoxy en $C_1$-$C_4$, alkyl($C_1$-$C_4$)-thio, halogénoalcoxy en $C_1$-$C_4$, halogénoalkyle en $C_1$-$C_4$ ou un radical $-CO(O)_m-R_{10}$, -COA, $-CON(A)R_3$ ou $-PO(R_5)R_6$, $R_1$ est en outre un radical alcynyle en $C_3$-$C_8$ qui n'est pas substitué ou un radical $-(E)_mU$ ou $-(E)_m-Q$, $R_2$ est un radical alkyle en $C_1$-$C_8$ ou cycloalkyle en $C_3$-$C_8$ qui n'est pas substitué ou est substitué par des substituants alcoxy en $C_1$-$C_8$, alkyl($C_1$-$C_8$)-thio, alcoxy($C_3$-$C_8$)-alcoxy, halogène, cyano, ou un radical $-CX(X)_mR_{10}$, $-(X)_m-CX-A$, $-(X)_mCXR_{10}$, $N(R_3)COA$, $-[N(R_3)]_m-C(A)=NH$, $-[N(R_3)]_m-C(A)=N$-alkyle($C_1$-$C_4$), A, -X-U ou -XQ, $R_2$ représente en outre un radical alcényle en $C_3$-$C_8$ ou cycloalcényle en $C_3$-$C_8$ qui n'est pas substitué ou est substitué par un halogène, ou un radical $-(E)_mU$ ou $-(E)_mQ$;

m est 0 ou 1;

Y est un atome d'oxygène, de soufre, SO ou $SO_2$;

A est un radical $-N(R_3)R_4$;

D est un radical $-N(R_3)N(R_4)(CO)_mR_3$;

$R_3$ et $R_4$ sont chacun, indépendamment l'un de l'autre, un atome d'hydrogène ou un radical alkyle en $C_1$-$C_8$ ou cycloalkyle en $C_3$-$C_8$ qui n'est pas substitué ou est substitué par un groupe alcoxy en $C_1$-$C_8$, alcoxy($C_2$-$C_8$)-alcoxy, alkyl($C_1$-$C_8$)-thio, cyano, $-COOR_{10}$, alkyl($C_1$-$C_4$)-carbamoyle, dialkyl($C_1$-$C_4$)-carbamoyle, alkyl($C_1$-$C_4$)-amino, dialkyl($C_1$-$C_4$)-amino, pipéridinocarbonyle, pyrrolidinocarbonyle, pipéridino ou pyrrolidino; $R_3$ et $R_4$ sont en outre un radical alcényle en $C_3$-$C_8$ ou cycloalcényle en $C_3$-$C_8$, qui n'est pas substitué ou est substitué par un atome d'halogène ou par un groupe alcoxy en $C_1$-$C_8$, cycloalcényle en $C_3$-$C_8$, cyano, $-COOR_{10}$, alkyl($C_1$-$C_4$)-carbamoyle, dialkyl($C_1$-$C_4$)-carbamoyle, U, pyrrolidinocarbamoyle ou pipéridinocarbamoyle, $R_3$ et $R_4$ sont en outre un radical alcynyle en $C_3$-$C_8$ qui n'est pas substitué ou est substitué par U, ou $R_3$ et $R_4$ représentent un radical $-(E)_m-U$ ou $-(E)_m-Q$.

$R_3$ et $R_4$ forment ensemble, avec l'atome d'azote auquel ils sont liés, également un hétérocycle saturé ou insaturé à 5-9 chaînons, qui peut être une ou plusieurs fois interrompu par un ou des atomes d'oxygène, de soufre, d'azote ou groupes -NH-, -N-alkyle($C_1$-$C_4$),

-CO- ou $-C(OR_7)OR_8-$ et substitué par un ou des atomes d'halogène ou groupes cyano, alcoxy en $C_1$-$C_8$, amino, alkyl($C_1$-$C_4$)-amino, dialkyl($C_1$-$C_4$)-amino ou $-COOR_{10}$;

$R_5$ et $R_6$ sont chacun, indépendamment l'un de l'autre, un groupe hydroxy, alkyle en $C_1$-$C_4$ ou alcoxy en $C_2$-$C_4$;

$R_7$ et $R_8$ sont chacun, indépendamment l'un de l'autre, un groupe alkyle en $C_1$-$C_4$, ou

$R_7$ et $R_8$ forment ensemble une chaîne alkylène à 2-4 chaînons,

$R_9$ et $R_{10}$ sont chacun, indépendamment l'un de l'autre, un atome d'hydrogène ou un groupe alkyle en $C_1$-$C_3$, cycloalkyle en $C_3$-$C_8$, alcényle en $C_3$-$C_8$, cycloalcényle en $C_3$-$C_8$, alcynyle en $C_3$-$C_8$, alcoxy($C_2$-$C_8$)-alkyle, alcoxy($C_4$-$C_8$)-alcoxyalkyle, halogénoalkyle en $C_1$-$C_4$, -[alkyl($C_1$-$C_3$)]$_m$U, -[alkyl($C_1$-$C_3$)]$_m$Q, halogénoalcoxy en $C_1$-$C_4$, halogénoalcoxy-($C_1$-$C_4$)-alkyle($C_1$-$C_4$);

U est un radical phényle ou naphtyle qui n'est pas substitué ou est substitué une ou plusieurs fois par un ou des substituants halogène, alkyle en $C_1$-$C_4$, -Y-alkyle($C_1$-$C_4$), halogénoalkyle en $C_1$-$C_4$, halogénoalcoxy en $C_1$-$C_4$, cyano, nitro, -COOH, $-COOR_7$, $-CONH_2$, $-CONHR_7$, $-CON(R_7)_2$, $SO_2NHR_7$, $SO_2N(R_7)_2$, pyrrolidino, pipéridino, pyrrolidinocarbonyle ou pipéridinocarbonyle;

E est une chaîne alkylène en $C_1$-$C_8$, alcénylène en $C_2$-$C_8$, qui n'est pas substituée ou est substituée par un atome d'halogène ou par un groupe alcoxy en $C_1$-$C_4$, alkyl($C_1$-$C_4$)-thio, halogénoalcoxy en $C_1$-$C_4$, $-CO(O)_mR_{10}$, $(CO)_m$-A ou $-(CO)_mQ$, et/ou est interrompue par un chaînon -CO- ou $-C(OR_7)OR_8-$;

Q est un hétérocycle saturé ou insaturé à 5-12 chaînons, qui peut contenir 1-4 hétéroatomes ou un groupe sulfinyle ou sulfonyle, également conjointement avec 1-2 hétéroatomes, qui peut être interrompu par 1 ou 2 groupes carbonyle et soudé à un noyau benzénique,

pour le traitement ou la prévention d'une attaque de plantes cultivées par des micro-organismes pathogènes.

2. Utilisation selon la revendication 1 de dérivés d'acide 2-chloro-4-trifluorométhyl-thiazole-5-carboxylique de formule Ib

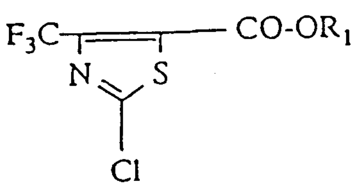

(Ib)

dans laquelle $R_1$ a la signification donnée dans la revendication 1.

3. Utilisation selon la revendication 1 de dérivés d'acide 2-chloro-4-trifluorométhyl-thiazole-5-carboxylique de formule Ib selon la revendication 2, dans lesquels $R_1$ représente le radical
tétrahydropyranne-2-ylméthyle,
2,2-diméthyl-1,3-dioxolanne-4-ylméthyle,
1,2-dihydrobenz-1,4-dioxanne-2-ylméthyle,
thiophène-2-ylméthyle,
3,4-méthylènedioxybenzyle,
5-méthylthiazole-4-yléthyle,
p-tolyl-éth-1-yle,
bornyle,
norbornyle,
fenchyle,
menthyle,
cyanoéthyle,
phénoxyéthyle,
méthylsulfonyléthyle,
phénylthioéthyle,
éthoxycarbonylméthyle,
éthoxycarbonyléth-1-yle,

5,5-diméthyl-tétrahydrofuranne-2-one-3-yle,
2-oxopyrrolinométhyle,
α-méthoxycarbonylbenzyle,
α-cyanobenzyle,
α-benzoylbenzyle ou
α-méthoxycarbonyl-α-phénylbenzyle ou
le radical morpholinométhyle.

4. Utilisation selon la revendication 1 de dérivés d'acide 2-chloro-4-trifluorométhyl- thiazole-5-carboxylique de formule Ib selon la revendication 2, dans lesquels $R_1$ représente un radical alkyle en $C_1$-$C_{12}$ qui est substitué par $-(X)_m$-CX-A, tandis que A, m et X ont les significations données dans la revendication 1.

5. Utilisation selon la revendication 1 de dérivés d'acide 2-chloro-4-trifluorométhyl-thiazole-5-carboxylique de formule Ib selon la revendication 4, dans lesquels m est zéro et X représente un atome d'hydrogène, tandis que A a la signification donnée dans la revendication 1.

6. Utilisation selon la revendication 1 de dérivés d'acide 2-chloro-4-trifluorométhyl-thiazole-5-carboxylique de formule Ib selon la revendication 2, dans lesquels $R_1$ représente le radical azépinométhyle, N-(2,6-diméthylphényl)-N-(méthoxycarbonyléth-1-yl)-carbamoylméthyle ou 2-pipéridino-éth-1-yle.

7. Utilisation selon la revendication 1 des dérivés d'acide 2-chloro-4-trifluorométhylthiazole-5-carboxylique de formule Ic

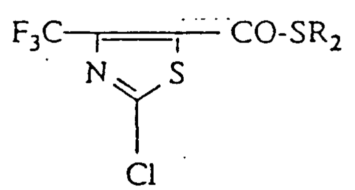

(Ic)

dans laquelle $R_2$ a la signification donnée dans la revendication 1, pour la lutte contre ou la prévention d'une attaque de plantes cultivées par des micro-organismes pathogènes.

8. Produit fongicide contenant, en tant que substance active, un dérivé d'acide 2-chloro-4-trifluorométhyl-thiazole-5-carboxylique de formule Id

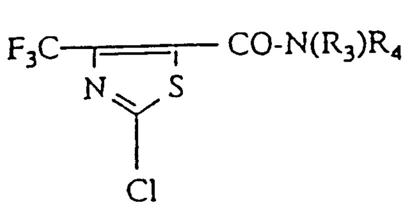

(Id)

dans laquelle l'un des radicaux $R_3$ et $R_4$ représente un atome d'hydrogène ou un groupe alkyle en $C_1$-$C_8$ non substitué, l'autre ou les deux représente(nt) un radical cycloalkyle en $C_3$-$C_8$ qui n'est pas substitué ou un radical alkyle en $C_1$-$C_8$ ou cycloalkyle en $C_3$-$C_8$ qui est substitué par un groupe alcoxy en $C_1$-$C_8$, alcoxy($C_2$-$C_8$)-alcoxy, alkyl($C_1$-$C_8$)-thio, cyano, $-COOR_{10}$, alkyl($C_1$-$C_4$)-carbamoyle, dialkyl-($C_1$-$C_4$)-carbamoyle, alkyl($C_1$-$C_4$)-amino, dialkyl($C_1$-$C_4$)-amino, pipéridinocarbonyle, pyrrolidinocarbonyle, pipéridino ou pyrrolidino; $R_3$ et $R_4$ sont en outre un radical alcényle en $C_3$-$C_8$ ou cycloalcényle en $C_3$-$C_8$ qui n'est pas substitué ou est substitué par un atome d'halogène ou un groupe alcoxy en $C_1$-$C_8$, cycloalcényle en $C_3$-$C_8$, cyano, $-COOR_{10}$, alkyl($C_1$-$C_4$)-carbamoyle, dialkyl($C_1$-$C_4$)-carbamoyle, U, pyrrolidinocarbamoyle, ou pipéridinocarbamoyle, $R_3$ et $R_4$ sont en outre un radical alcynyle en $C_3$-$C_8$ qui n'est pas substitué ou est substitué par U, ou $R_3$ et $R_4$ représentent un radical $-(E)_m$-U ou $-(E)_m$-Q, et $R_3$ et $R_4$ forment également, conjointement avec l'atome d'azote auquel ils sont liés, un hétérocycle saturé ou insaturé à 5-9 chaînons, qui peut être une ou plusieurs fois interrompu par des atomes d'oxygène, de soufre, d'azote ou des groupes -NH-, -N-alkyl($C_1$-$C_4$)-, -CO- ou $-C(OR_7)OR_8$- et substitué

par un ou des substituants halogène, cyano, alcoxy en $C_1$-$C_8$, amino, alkyl($C_1$-$C_4$)-amino, dialkyl($C_1$-$C_4$)-amino ou -$COOR_{10}$.

**9.** Utilisation selon la revendication 1 des dérivés d'acide 2-chloro-4-trifluorométhyl-thiazole-5-carboxylique de formule Id

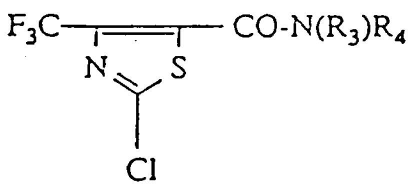

(Id)

dans laquelle $R_3$ et $R_4$ ont les significations données dans la revendication 8, pour la lutte contre ou la prévention d'une attaque de plantes cultivées par des micro-organismes pathogènes.

**10.** Procédé pour la lutte contre la prévention d'une attaque par des micro-organismes phytopathogènes de plantes cultivées, caractérisé en ce que l'on applique sur les plantes ou leur site un composé de formule Ia défini selon la revendication 1.

**11.** Utilisation de composés de formule I selon la revendication 1 ou de produits les contenant, pour la lutte contre et/ou la prévention d'une attaque par des micro-organismes.

**12.** Utilisation selon la revendication 11, caractérisée en ce que, en ce qui concerne les micro-organismes, il s'agit de champignons phytopathogènes.

**13.** Procédé selon la revendication 10, caractérisé en ce que les parties de plantes sont les graines.

**14.** Procédé selon la revendication 10, caractérisé en ce que l'on traite des plants de riz.

**15.** Utilisation selon la revendication 11, caractérisé en ce que, en ce qui concerne les micro-organismes, il s'agit de bactéries phytopathogènes du sol.